(19) Europäisches Patentamt / European Patent Office / Office européen des brevets

(11) **EP 3 034 070 A1**

(12) **EUROPEAN PATENT APPLICATION**

(43) Date of publication:
**22.06.2016 Bulletin 2016/25**

(51) Int Cl.:
*A61K 9/20* (2006.01)          *A61K 31/167* (2006.01)

(21) Application number: **14199037.4**

(22) Date of filing: **18.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Omya International AG**
**4665 Oftringen (CH)**

(72) Inventors:
• **Huwyler, Jörg**
  **4144 Arlesheim (CH)**

• **Puchkov, Maxim**
  **4148 Pfeffingen (CH)**
• **Stirnimann, Tanja**
  **4800 Zofingen (CH)**
• **Atria, Susanna**
  **6032 Emmen (CH)**

(74) Representative: **Boos, Melanie**
  **Maiwald Patentanwalts GmbH**
  **Elisenhof**
  **Elisenstraße 3**
  **80335 München (DE)**

(54) **Method for the production of a pharmaceutical delivery system**

(57)    The present invention relates to a method for producing a pharmaceutical delivery system, the use of a surface-reacted calcium carbonate for improving the friability of a pharmaceutical delivery system as well as for improving the flowability of a pharmaceutical delivery system and a pharmaceutical delivery system obtained by the method.

Fig. 1

EP 3 034 070 A1

## Description

The present invention relates to a method for producing a pharmaceutical delivery system, the use of a surface-reacted calcium carbonate for improving the friability of a pharmaceutical delivery system as well as for improving the flowability of a pharmaceutical delivery system and a pharmaceutical delivery system obtained by the method.

Pharmaceutical delivery systems are designed to release pharmaceutical active agents to the human or animal body. Numerous carriers have been used in such systems including waxes, oils, fats, soluble polymers, and the like. Another approach provides to disperse the pharmaceutical active agent throughout a solid matrix material through which said active agent is released by diffusion. Still another approach provides to enclose the pharmaceutical active agent within a capsule having polymeric walls through which said active agent can pass by diffusion.

For example, Pawar et al. (Gastroretentive dosage forms: A review with special emphasis on floating drug delivery systems. Drug Delivery. 2011 Feb;18(2):97-110) considered floating drug delivery systems (FDDS) as an easy and logical approach regarding formulation and technical aspects for the development of GRDDS.

US 3,976,764 discloses an instantly floating tablet, having a hollow sphere based on gelatin coated with several under-coatings, wherein an therapeutically active ingredient is comprised in one of the under-coatings.

DE 35 27 852 A1 discloses a pharmaceutical formulation with a specific density below 1, wherein a substance forming a gel in water is mixed with a pharmaceutically active agent and a fat/oil which is solid at room temperature. The gel forming substance being a derivative of cellulose, dextran or starch.

EP 0 338 861 A2, refers to an antacid compositions with prolonged gastric residence time. The antacid such as Hydrotalcite or Amalgate forming a solid core which is surrounded by a solid external phase containing a hydrophobic substance e.g. an ester of glycerol with palmitic or stearic acid, hydroxylated polyalkene and a nonionic emulsifier.

EP 0 717 988 A1, refers to a swollen moulding which is an expanded structure having a mesh-like cross-section and an apparent density of less than 1, which structure is predominantly an acid-resistant polymer compound and additionally containing at least an auxiliary blowing agent and a drug substance. Because of its mesh-like structure in cross-section, the swollen moulding of the invention has a multiplicity of microfine internal pores which are continuous or discontinuous. Said acid-resistant polymer compound are chosen, e.g. from hydroxpropymethylcellulose acetate succinate or phthalate.

US 4,451,260 refers to a multilayered structure comprising a pharmaceutical active ingredient wherein air is entrapped in the multilayered structure, thus promoting flotation.

US 4,814,179 refers to a floating sustained release therapeutic composition. Non-compressed sustained release tablets comprise a hydrocolloid gelling agent, a therapeutically acceptable inert oil, the selected therapeutic agent and water.

EP 2 719 376 A1 refers to gastroretentive drug formulations and delivery systems using functionalized calcium carbonate and their method of preparation.

In this regard, calcium carbonate seems to be promising for the preparation of pharmaceutical delivery system as it exhibits a highly porous meshwork with lamellar surface structure that grips particles strongly together, see e.g. EP 2 719 373 A1. It thus offers the possibility to formulate them in to granules, pellets, capsules or to compact them into tablets or mini-tablets.

A new type of surface-reacted calcium carbonate was first described in FR 2787802 B1 in the year of 1998, subsequently in WO 00/39222 A1 and US 2004/0020410 A1, and is based on the reaction of natural ground calcium carbonate with gaseous $CO_2$ and with one or more medium-strong to strong $H_3O^+$ ion providers. The obtained product is a porous calcium carbonate having a special surface structure, porosity, and specific surface area providing a reduction in the weight of paper for a constant surface area without loss of physical properties, when it is used as a pigment or coating filler for the said paper.

In WO 2004/083316 A1, a further advantageous modification in the preparation of this surface-reacted calcium carbonate is described, wherein aluminium silicate, synthetic silica, calcium silicate, silicates and/or monovalent salt are involved, and which are also useful in paper-making applications. Also, WO 2005/121257 A2 refers to the addition of advantageous additives in the production of said surface-reacted calcium carbonate, wherein one or more compounds of formula R-X are added, which, e.g. are selected from fatty acids, fatty amines or fatty alcohols. WO 2009/074492 A1 especially relates to the optimization of the known process as regards precipitated calcium carbonate, as it turned out that due to the special conditions in the precipitation of calcium carbonate, the process useful for natural ground calcium carbonate did not provide the same good results for the surface-reaction of synthetic precipitated calcium carbonate. Several further optimizations and modifications of the process for the preparation of surface-reacted calcium carbonate followed such as those described in EP 2 264 108 A1 (WO 2010/146530 A1) and EP 2 264 109 A1 (WO 2010/146531 A1) involving the use of weak acids in the preparation of surface-reacted calcium carbonate.

WO2014/001063 relates to a high solids aqueous mineral filler suspension which maintains its mechanical properties of a suspension at low temperatures and acidic environment.

However, none of the foregoing documents explicitly mention efficient compacting methods for producing pharmaceutical delivery systems comprising surface-reacted calcium carbonate.

Thus, there is a continuous need for pharmaceutical delivery systems and methods for their production which provide a better performance than existing pharmaceutical delivery systems and especially allows for producing a pharmaceutical delivery system having improved friability and/or flowability as compared to a conventional pharmaceutical delivery systems using microcrystalline cellulose instead of surface-reacted calcium carbonate. Furthermore, it is desired to provide methods for producing pharmaceutical delivery systems which are efficient and allows for sufficient compacting of the system.

It is thus an object of the present invention to provide a method for producing a pharmaceutical delivery system. Another object may also be seen in the provision of a highly efficient compacting method for producing a pharmaceutical delivery system. A further object may be seen in the provision of a method for producing a pharmaceutical delivery system having improved friability and/or flowability as compared to a conventional pharmaceutical delivery system using microcrystalline cellulose instead of surface-reacted calcium carbonate.

One or more of the foregoing and other problems are solved by the subject-matter as defined herein in the independent claims. Advantageous embodiments of the present invention are defined in the corresponding sub-claims.

A first aspect of the present invention relates to a method for producing a pharmaceutical delivery system. The method comprising the steps of:

a) providing surface-reacted calcium carbonate, which is a reaction product of natural ground or precipitated calcium carbonate with carbon dioxide and one or more acids in an aqueous medium, wherein the carbon dioxide is formed in situ by the acid treatment and/or is supplied from an external source;
b) providing at least one pharmaceutically active agent or pharmaceutically inactive precursor thereof;
c) providing at least one formulating aid;
d) mixing the surface-reacted calcium carbonate of step a), the at least one pharmaceutically active agent or pharmaceutically inactive precursor thereof of step b) and the at least one formulating aid of step c); and
e) compacting the mixture obtained in step d) by means of a roller compacter at a compaction pressure in the range from 4 to 20 bar; and
f) compacting the roller compacted mixture obtained in step e) for obtaining the pharmaceutical delivery system.

According to another aspect of the present invention, the use of a surface-reacted calcium carbonate for improving the friability of a pharmaceutical delivery system is provided, wherein the surface-reacted calcium carbonate is a reaction product of natural ground or precipitated calcium carbonate with carbon dioxide and one or more acids in an aqueous medium, wherein the carbon dioxide is formed in situ by the acid treatment and/or is supplied from an external source, wherein the pharmaceutical delivery system a) has a friability of $\leq 1.10\,\%$, measured according to Ph.Eur.4 on tablets compressed at 100 MPa; and/or b) fulfils Formula (II)

$$(FR_{RCPh}) / (FR_{RCPl}) \geq 1.2 \qquad (I)$$

wherein

$(FR_{RCPh})$ is the friability (in %), measured according to Ph.Eur.4, of a pharmaceutical delivery system RCPh, wherein the compaction pressure in step e) is at least 15 bar; $(FR_{RCPl})$ is the friability (in %), measured according to Ph.Eur.4, of a pharmaceutical delivery system RCPl, wherein the compaction pressure in step e) is less than the compaction pressure used for obtaining the pharmaceutical delivery system RCPh.

According to a further aspect of the present invention, the use of a surface-reacted calcium carbonate for improving the flowability of a pharmaceutical delivery system is provided, wherein the surface-reacted calcium carbonate is a reaction product of natural ground or precipitated calcium carbonate with carbon dioxide and one or more acids in an aqueous medium, wherein the carbon dioxide is formed in situ by the acid treatment and/or is supplied from an external source, wherein the pharmaceutical delivery system fulfils Formula (II)

$$(FL_{RCP}) / (FL_{reference}) \geq 1.1 \qquad (II)$$

wherein

$(FL_{RCP})$ is the flowability (in g/s), measured according to Ph.Eur.4 at an opening diameter of 7 mm, of the pharmaceutical delivery system;

(FL$_{reference}$) is the flowability (in g/s), measured according to Ph.Eur.4 at an opening diameter of 7 mm, of the same pharmaceutical delivery system, in which the surface-reacted calcium carbonate is replaced by microcrystalline cellulose.

According to a still further aspect of the present invention, a pharmaceutical delivery system, preferably a tablet, mini-tablet, capsule or pellet, obtained by the method is provided.

According to one embodiment of the present method, the natural ground calcium carbonate is selected from calcium carbonate containing minerals selected from the group comprising marble, chalk, dolomite, limestone and mixtures thereof; or the precipitated calcium carbonate is selected from the group comprising precipitated calcium carbonates having aragonitic, vateritic or calcitic mineralogical crystal forms and mixtures thereof.

According to another embodiment of the present method, the surface-reacted calcium carbonate a) has a BET specific surface area of from 20.0 m²/g to 200.0 m²/g, preferably from 20.0 m²/g to 180.0 m²/g, more preferably from 30.0 m²/g to 160.0 m²/g, even more preferably from 40.0 m²/g to 150.0 m²/g, and most preferably from 50.0 m²/g to 140.0 m²/g, measured using nitrogen and the BET method according to ISO 9277; and/or b) comprises particles having a volume median grain diameter $d_{50}$ of from 2.0 to 50.0 μm, preferably from 2.5 to 25.0 μm, more preferably from 2.8 to 20.0 μm, even more preferably from 3.0 to 10.0 μm, and most preferably from 4.0 to 8.0 μm; and/or c) has an intra-particle intruded specific pore volume within the range of 0.15 to 1.35 cm³/g, preferably of 0.30 to 1.30 cm³/g, and most preferably of 0.40 to 1.25 cm³/g, calculated from a mercury intrusion porosimetry measurement.

According to yet another embodiment of the present method, the at least one pharmaceutically active agent or pharmaceutically inactive precursor thereof is selected from the group comprising pharmaceutically active agent or pharmaceutically inactive precursor of synthetic origin, semi-synthetic origin, natural origin and combinations thereof.

According to one embodiment of the present method, the at least one formulating aid a) is at least one inner-phase lubricant and/or outer-phase lubricant; and/or b) is provided in a total amount from about 0.1 wt.-% to about 10.0 wt.-%, preferably from about 0.3 wt.-% to about 5.0 wt%, more preferably from about 0.5 wt.-% to about 2.5 wt.-% based on the total weight of the pharmaceutical delivery system.

According to another embodiment of the present method, roller compacting step e) is carried out at a roller compaction pressure in the range from 4 to 15 bar, more preferably in the range from 4 to 10 bar and most preferably in the range from 4 to 7 bar.

According to yet another embodiment of the present method, compacting step f) is a pelletizing or tableting step.

According to one embodiment of the present method, the roller compacted mixture obtained in roller compacting step e) is subjected to a milling step before compacting step f) is carried out.

According to another embodiment of the present method, the roller compacted mixture has a grain size of from 180 to 710 μm obtained by sieving on different mesh sizes, preferably with mesh sizes of 180 μm, 250 μm, 355 μm, 500 μm and 710 μm.

According to yet another embodiment of the present method, the pharmaceutical delivery system a) has a friability of ≤ 1.10 %, measured according to Ph.Eur.4 on tablets compressed at 100 MPa; and/or b) fulfils Formula (I)

$$(FR_{RCPh}) / (FR_{RCPl}) \geq 1.2 \quad (I)$$

wherein

(FR$_{RCPh}$) is the friability (in %), measured according to Ph.Eur.4, of a pharmaceutical delivery system RCPh, wherein the compaction pressure in step e) is at least 15 bar; (FR$_{RCPl}$) is the friability (in %), measured according to Ph.Eur.4, of a pharmaceutical delivery system RCPl, wherein the compaction pressure in step e) is less than the compaction pressure used for obtaining the pharmaceutical delivery system RCPh.

According to one embodiment of the present method, the pharmaceutical delivery system fulfils Formula (II)

$$(FL_{RCP}) / (FL_{reference}) \geq 1.1 \quad (II)$$

wherein

(FL$_{RCP}$) is the flowability (in g/s), measured according to Ph.Eur.4 at an opening diameter of 7 mm, of the pharmaceutical delivery system;

(FL$_{reference}$) is the flowability (in g/s), measured according to Ph.Eur.4 at an opening diameter of 7 mm, of the same pharmaceutical delivery system, in which the surface-reacted calcium carbonate is replaced by microcrystalline cellulose.

According to another embodiment of the present method, the pharmaceutical delivery system is a tablet, mini-tablet, capsule or pellet.

It should be understood that for the purpose of the present invention the following terms have the following meaning.

For the purpose of the present invention, an "acid" is defined as Bronsted-Lowry acid, that is to say, it is an $H_3O^+$ ion provider. An "acid salt" is defined as an $H_3O^+$ ion-provider, e.g., a hydrogen-containing salt, which is partially neutralised by an electropositive element. A "salt" is defined as an electrically neutral ionic compound formed from anions and cations. A "partially crystalline salt" is defined as a salt that, on XRD analysis, presents an essentially discrete diffraction pattern.

In accordance with the present invention, $pK_a$ is the symbol representing the acid dissociation constant associated with a given ionisable hydrogen in a given acid, and is indicative of the natural degree of dissociation of this hydrogen from this acid at equilibrium in water at a given temperature. Such $pK_a$ values may be found in reference textbooks such as Harris, D. C. "Quantitative Chemical Analysis: 3rd Edition", 1991, W.H. Freeman & Co. (USA), ISBN 0-7167-2170-8.

"Natural ground calcium carbonate" (GCC) in the meaning of the present invention is a calcium carbonate obtained from natural sources, such as limestone, marble, dolomite, or chalk, and processed through a wet and/or dry treatment such as grinding, screening and/or fractionating, for example, by a cyclone or classifier.

"Precipitated calcium carbonate" (PCC) in the meaning of the present invention is a synthesised material, obtained by precipitation following reaction of carbon dioxide and lime in an aqueous, semi-dry or humid environment or by precipitation of a calcium and carbonate ion source in water. PCC may be in the vateritic, calcitic or aragonitic crystal form.

For the purpose of the present invention, a "surface-reacted calcium carbonate" is a material comprising calcium carbonate and a water insoluble, at least partially crystalline, non-carbonate calcium salt, preferably, extending from the surface of at least part of the calcium carbonate. The calcium ions forming said at least partially crystalline non-carbonate calcium salt originate largely from the starting calcium carbonate material that also serves to form the surface-reacted calcium carbonate core. Such salts may include OH$^-$ anions and/or crystal water.

In the meaning of the present invention "water-insoluble" materials are defined as materials which, when mixed with deionised water and filtered on a filter having a 0.2 $\mu$m pore size at 20°C to recover the liquid filtrate, provide less than or equal to 0.1 g of recovered solid material following evaporation at 95 to 100°C of 100 g of said liquid filtrate. "Water-soluble" materials are defined as materials leading to the recovery of greater than 0.1 g of recovered solid material following evaporation at 95 to 100°C of 100 g of said liquid filtrate.

Throughout the present document, the "particle size" of a calcium carbonate and other materials is described by its distribution of particle sizes. The value $d_x$ represents the diameter relative to which x % by weight of the particles have diameters less than $d_x$. This means that the $d_{20}$ value is the particle size at which 20 wt.-% of all particles are smaller, and the $d_{75}$ value is the particle size at which 75 wt.-% of all particles are smaller. The $d_{50}$ value is thus the weight median particle size, i.e. 50 wt.-% of all grains are bigger and the remaining 50 wt.-% of grains smaller than this particle size. For the purpose of the present invention the particle size is specified as weight median particle size $d_{50}$ unless indicated otherwise. For determining the weight median particle size $d_{50}$ value a Sedigraph can be used. For the purpose of the present invention, the "particle size" of surface-reacted calcium carbonate is described as volume determined particle size distributions. For determining the volume determined particle size distribution, e.g., the volume median grain diameter ($d_{50}$) or the volume determined top cut particle size ($d_{98}$) of surface-reacted calcium carbonate, a Malvern Mastersizer 2000 can be used. The weight determined particle size distribution may correspond to the volume determined particle size if the density of all the particles is equal.

A "specific surface area (SSA)" of a calcium carbonate in the meaning of the present invention is defined as the surface area of the calcium carbonate divided by its mass. As used herein, the specific surface area is measured by nitrogen gas adsorption using the BET isotherm (ISO 9277:2010) and is specified in $m^2$/g.

For the purpose of the present invention the "porosity" or "pore volume" refers to the intra-particle intruded specific pore volume. The porosity or pore volume is measured using a Micromeritics Autopore IV 9500 mercury porosimeter having a maximum applied pressure of 414 MPa, equivalent to a Laplace throat diameter of 0.004 $\mu$m.

A "suspension" or "slurry" in the meaning of the present invention comprises insoluble solids and water, and optionally further additives, and usually contains large amounts of solids and, thus, is more viscous and can be of higher density than the liquid from which it is formed.

The term "compacting" in the meaning of the present invention means a reduction in porosity and an increase of the tablet hardness which is obtained under pressure.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably. This e.g. means that, unless the context clearly dictates otherwise, the term "obtained" does not mean to indicate that e.g. an embodiment must be obtained by e.g. the sequence of steps following the term "obtained" though such a limited understanding is always included by the terms "obtained" or "defined" as a preferred embodiment.

According to the present invention it has been found that calcium carbonate, which has been surface treated in a certain way, can be used as excipient in pharmaceutical delivery systems and provides the possibility of efficiently compacting the pharmaceutical delivery systems.

In the following, it is referred to further details of the present invention and especially the foregoing steps of the method for producing a pharmaceutical delivery system.

According to step a) of the instant method, a surface-reacted calcium carbonate is provided, which is a reaction product of natural ground or precipitated calcium carbonate with carbon dioxide and one or more acids in an aqueous medium, wherein the carbon dioxide is formed in situ by the acid treatment and/or is supplied from an external source.

The surface-reacted calcium carbonate used according to the present invention is a reaction product of natural ground or precipitated calcium carbonate with carbon dioxide and at least one acid in an aqueous medium, wherein the carbon dioxide is formed in situ by the acid treatment and/or is supplied from an external source.

The expression "acid treatment" in the meaning of the present invention refers to the reaction of the natural ground or precipitated calcium carbonate and the at least one acid in the aqueous medium. By this reaction carbon dioxide can be formed in-situ in the aqueous medium.

Natural ground calcium carbonate (GCC) is understood to be a naturally occurring form of calcium carbonate, mined from sedimentary rocks such as limestone or chalk, or from metamorphic marble rocks. Calcium carbonate is known to exist mainly as three types of crystal polymorphs: calcite, aragonite and vaterite. Calcite, the most common crystal polymorph, is considered to be the most stable crystal form of calcium carbonate. Less common is aragonite, which has a discrete or clustered needle orthorhombic crystal structure. Vaterite is the rarest calcium carbonate polymorph and is generally unstable. Natural calcium carbonate is almost exclusively of the calcitic polymorph, which is said to be trigonal-rhombohedral and represents the most stable of the calcium carbonate polymorphs. The term "source" of the calcium carbonate in the meaning of the present invention refers to the naturally occurring mineral material from which the calcium carbonate is obtained. The source of the calcium carbonate may comprise further naturally occurring components such as magnesium carbonate, alumino silicate etc.

According to one embodiment the natural ground calcium carbonate is selected from calcium carbonate containing minerals selected from the group consisting of marble, chalk, dolomite, limestone, and mixtures thereof.

According to one embodiment of the present invention the GCC is obtained by dry grinding. According to another embodiment of the present invention the GCC is obtained by wet grinding and optionally subsequent drying.

In general, the grinding step can be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. It is to be noted that the same grinding methods can be used for dry grinding the calcium carbonate containing mineral material. The wet processed ground calcium carbonate containing mineral material thus obtained may be washed and dewatered by well-known processes, e.g. by flocculation, filtration or forced evaporation prior to drying. The subsequent step of drying may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and lime in an aqueous environment or by precipitation of a calcium and carbonate ion source in water or by precipitation of calcium and carbonate ions, for example $CaCl_2$ and $Na_2CO_3$, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the precipitated calcium carbonate is selected from the group consisting of precipitated calcium carbonates having aragonitic, vateritic or calcitic mineralogical crystal forms, and mixtures thereof.

In a preferred embodiment, the natural ground or precipitated calcium carbonate is ground prior to the treatment with the at least one acid and carbon dioxide. The grinding step can be carried out with any conventional grinding device such as a grinding mill known to the skilled person.

In a preferred process, the natural ground or precipitated calcium carbonate, either finely divided, such as by grinding, or not, is suspended in water. Preferably, the slurry has a content of natural ground or precipitated calcium carbonate within the range of 1 wt.-% to 80 wt.-%, more preferably 3 wt.-% to 60 wt.-%, and even more preferably 5 wt.-% to 40 wt.-%, based on the weight of the slurry.

According to one embodiment of the present invention, the natural ground or precipitated calcium carbonate has a weight median particle size $d_{50}$ of from 0.1 to 50 $\mu$m, preferably from 0.5 to 25 $\mu$m, more preferably from 0.8 to 20 $\mu$m, even more preferably from 1.0 to 10 $\mu$m and most preferably from 1.2 to 8 $\mu$m

In a next step, at least one acid is added to the aqueous suspension containing the natural ground or precipitated calcium carbonate. The at least one acid can be any strong acid, medium-strong acid, or weak acid, or mixtures thereof, generating $H_3O^+$ ions under the preparation conditions. According to the present invention, the at least one acid can also be an acidic salt, generating $H_3O^+$ ions under the preparation conditions.

According to one embodiment, the at least one acid is a strong acid having a $pK_a$ of 0 or less at 20°C. According to another embodiment, the at least one acid is a medium-strong acid having a $pK_a$ value from 0 to 2.5 at 20°C. If the $pK_a$ at 20°C is 0 or less, the acid is preferably selected from sulphuric acid, hydrochloric acid, or mixtures thereof. If the $pK_a$ at 20°C is from 0 to 2.5, the acid is preferably selected from $H_2SO_3$, $H_3PO_4$, oxalic acid, or mixtures thereof. According to a preferred embodiment, the least one acid is $H_3PO_4$. The at least one acid can also be an acidic salt, for example, $HSO_4^-$ or $H_2PO_4^-$, being at least partially neutralized by a corresponding cation such as $Li^+$, $Na^+$ or $K^+$, or $HPO_4^{2-}$, being at least partially neutralised by a corresponding cation such as $Li^+$, $Na^+$, $K^+$, $Mg^{2+}$ or $Ca^{2+}$. The at least one acid can also be a mixture of one or more acids and one or more acidic salts.

According to still another embodiment, the at least one acid is a weak acid having a $pK_a$ value of greater than 2.5 and less than or equal to 7, when measured at 20°C, associated with the ionisation of the first available hydrogen, and having a corresponding anion formed on loss of this first available hydrogen, which is capable of forming water-soluble calcium salts. According to the preferred embodiment, the weak acid has a $pK_a$ value from 2.6 to 5 at 20°C, and more preferably the weak acid is selected from the group consisting of acetic acid, formic acid, propanoic acid, and mixtures thereof.

In case a weak acid is used, after addition of said acid to the aqueous suspension containing the natural ground or precipitated calcium carbonate, at least one water-soluble salt, which in the case of a hydrogen-containing salt has a $pK_a$ of greater than 7, when measured at 20°C, associated with the ionisation of the first available hydrogen, and the salt anion of which is capable of forming water-insoluble calcium salts, is additionally added. The cation of said water-soluble salt is preferably selected from the group consisting of potassium, sodium, lithium and mixtures thereof. In a more preferred embodiment, said cation is sodium. It is of note that depending on the charge of the anion, more than one of said cations may be present to provide an electrically neutral ionic compound. The anion of said water-soluble salt is preferably selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of drop wise addition, this addition preferably takes place within a time period of 15 minutes. It is more preferred to add said salt in one step.

According to one embodiment of the present invention, the at least one acid is selected from the group consisting of hydrochloric acid, sulphuric acid, sulphurous acid, phosphoric acid, citric acid, oxalic acid, acetic acid, formic acid, and mixtures thereof. Preferably the at least one acid is selected from the group consisting of hydrochloric acid, sulphuric acid, sulphurous acid, phosphoric acid, oxalic acid, $H_2PO_4^-$, being at least partially neutralised by a corresponding cation such as $Li^+$, $Na^+$ or $K^+$, $HPO_4^{2-}$, being at least partially neutralised by a corresponding cation such as $Li^+$, $Na^+$, $K^+$, $Mg^{2+}$ or $Ca^{2+}$, and mixtures thereof, more preferably the at least one acid is selected from the group consisting of hydrochloric acid, sulphuric acid, sulphurous acid, phosphoric acid, oxalic acid, or mixtures thereof, and most preferably, the at least one acid is phosphoric acid.

According to another embodiment of the present invention, the at least one acid is a mixture of one or more acids. For example, the at least one acid is a mixture of phosphoric acid and citric acid. The one or more acids may be added simultaneously or successively.

The at least one acid can be added to the suspension as a concentrated solution or a more diluted solution. According to one embodiment, the molar ratio of the at least one acid to the natural ground or precipitated calcium carbonate is from 0.01 to 0.6, preferably from 0.05 to 0.55, and more preferably from 0.1 to 0.5. As an alternative, it is also possible to add the at least one acid to the water before the natural ground or precipitated calcium carbonate is suspended.

In a next step, the natural ground or precipitated calcium carbonate is treated with carbon dioxide. The carbon dioxide can be formed in situ by the acid treatment and/or can be supplied from an external source. If a strong acid such as sulphuric acid or hydrochloric acid or a medium-strong acid such as phosphoric acid is used for the acid treatment of the natural ground or precipitated calcium carbonate, the carbon dioxide is automatically formed in a sufficient amount to achieve the required molar concentration. Alternatively or additionally, the carbon dioxide can be supplied from an external source.

According to one embodiment, the surface-reacted calcium carbonate is a reaction product of natural ground or precipitated calcium carbonate with carbon dioxide and at least one acid, wherein the carbon dioxide is formed in situ as a result of contacting the at least one acid with the natural ground or precipitated calcium carbonate and/or is supplied from an external source.

Acid treatment and treatment with carbon dioxide can be carried out simultaneously which is the case when a strong or medium-strong acid is used. It is also possible to carry out acid treatment first, e.g. with a medium strong acid having a $pK_a$ in the range of 0 to 2.5 at 20°C, wherein carbon dioxide is formed in situ, and thus, the carbon dioxide treatment will automatically be carried out simultaneously with the acid treatment, followed by the additional treatment with carbon dioxide supplied from an external source.

Preferably, the concentration of gaseous carbon dioxide in the suspension is, in terms of volume, such that the ratio (volume of suspension):(volume of gaseous $CO_2$) is from 1:0.05 to 1:20, even more preferably from 1:0.05 to 1:5.

In a preferred embodiment, the acid treatment step and/or the carbon dioxide treatment step are repeated at least once, more preferably several times.

Subsequent to the acid treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted natural ground or precipitated calcium carbonate as an aqueous suspension having a pH of greater than 6.0, preferably greater than 6.5, more preferably greater than 7.0, even more preferably greater than 7.5. If the aqueous suspension is allowed to reach equilibrium, the pH is greater than 7. A pH of greater than 6.0 can be adjusted without the addition of a base when stirring of the aqueous suspension is continued for a sufficient time period, preferably 1 hour to 10 hours, more preferably 1 to 5 hours.

Alternatively, prior to reaching equilibrium, which occurs at a pH greater than 7, the pH of the aqueous suspension may be increased to a value greater than 6 by adding a base subsequent to carbon dioxide treatment. Any conventional base such as sodium hydroxide or potassium hydroxide can be used.

Further details about the preparation of the surface-reacted calcium carbonate are disclosed in WO 00/39222, WO 2004/083316, WO 2005/121257, WO 2009/074492, EP 2 264 108, EP 2 264 109 and US 2004/0020410.

Similarly, surface-reacted precipitated calcium carbonate is obtained. As can be taken in detail from EP 2 070 991, surface-reacted precipitated calcium carbonate is obtained by contacting precipitated calcium carbonate with $H_3O^+$ ions and with anions being solubilized in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by $H_3O^+$ ions, where said $H_3O^+$ ions are provided solely in the form of a counterion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said $H_3O^+$ ions may be provided by the addition of an acid or an acid salt of said anion, or the addition of an acid or an acid salt which simultaneously serves to provide all or part of said excess solubilized calcium ions.

According to one embodiment of the present invention, the surface-reacted calcium carbonate is obtained by a process comprising the steps of:

a) providing a suspension of natural ground or precipitated calcium carbonate,
b) adding at least one acid having a $pK_a$ value of 0 or less at 20°C or having a $pK_a$ value from 0 to 2.5 at 20°C to the suspension of step a), and
c) treating the suspension of step a) with carbon dioxide before, during or after step b).

According to one embodiment, at least one acid having a $pK_a$ value of 0 or less at 20°C is added in step b) to the suspension of step a). According to another embodiment, at least one acid having a $pK_a$ value from 0 to 2.5 at 20°C is added in step b) to the suspension of step a).

The carbon dioxide used in step c) can be formed in situ by the acid treatment of step b) and/or can be supplied from an external source.

According to one embodiment of the present invention, the surface-reacted calcium carbonate is obtained by a process comprising the steps of:

A) providing a natural ground or precipitated calcium carbonate,
B) providing at least one water-soluble acid,
C) providing gaseous $CO_2$,
D) contacting said natural ground or precipitated calcium carbonate of step A) with the at least one acid of step B) and with the $CO_2$ of step C),

characterised in that:

i) the at least one acid of step B) has a $pK_a$ of greater than 2.5 and less than or equal to 7 at 20°C, associated with the ionisation of its first available hydrogen, and a corresponding anion is formed on loss of this first available hydrogen capable of forming a water-soluble calcium salt, and
ii) following contacting the at least one acid with natural ground or precipitated calcium carbonate, at least one water-soluble salt, which in the case of a hydrogen-containing salt has a $pK_a$ of greater than 7 at 20°C, associated with the ionisation of the first available hydrogen, and the salt anion of which is capable of forming water-insoluble calcium salts, is additionally provided.

According to one embodiment of the preparation of the surface-reacted natural ground or precipitated calcium carbonate, the natural ground or precipitated calcium carbonate is reacted with the at least one acid and/or the carbon dioxide in the presence of at least one compound selected from the group consisting of silicate, magnesium oxide, citric acid, aluminium sulphate, aluminium nitrate, aluminium chloride, and mixtures thereof. These components can be added to an aqueous suspension comprising the natural ground or precipitated calcium carbonate before adding the at least one acid and/or carbon dioxide.

The surface-reacted natural or synthetic calcium carbonate can be kept in suspension. Alternatively, the aqueous suspension described above can be dried.

The surface-reacted natural or precipitated calcium carbonate to be used in the present invention preferably is provided in the dried powder form. The subsequent step of drying may be carried out by any method known to the skilled person. For example, the drying can be carried out in a single step such as spray drying, or in at least two steps, e.g. by applying a first heating step to the surface-reacted calcium carbonate in order to reduce the associated moisture content, and applying a second heating step to the surface-reacted calcium carbonate in order to reduce the remaining moisture content.

The term "dry" surface-reacted calcium carbonate is understood to be a surface-reacted calcium carbonate having less than 1.8 % by weight of water relative to the surface-reacted calcium carbonate weight. The % water may be determined by heating the surface-reacted calcium carbonate to 105°C in a drying chamber using the method according to ISO 787-2.

The surface-reacted natural ground or precipitated calcium carbonate to be used in the present invention is provided in dry form. The surface-reacted calcium carbonate is preferably in the form of dust or powder and most preferably in the form of powder.

According to one embodiment of the present invention, the surface-reacted calcium carbonate comprises an insoluble, at least partially crystalline calcium salt of an anion of the at least one acid, which is formed on the surface of the natural ground or precipitated calcium carbonate. According to one embodiment, the insoluble, at least partially crystalline salt of an anion of the at least one acid covers the surface of the natural ground or precipitated calcium carbonate at least partially, preferably completely. Depending on the employed at least one acid, the anion may be sulphate, sulphite, phosphate, citrate, oxalate, acetate and/or formiate.

According to one preferred embodiment, the surface-reacted calcium carbonate is a reaction product of natural ground calcium carbonate and at least one acid, preferably phosphoric acid. According to another preferred embodiment, the surface-reacted calcium carbonate is a reaction product of natural ground calcium carbonate and phosphoric acid in combination with citric acid.

Furthermore, in a preferred embodiment, the surface-reacted calcium carbonate has a BET specific surface area of from 20.0 $m^2/g$ to 200.0 $m^2/g$, preferably from 20.0 $m^2/g$ to 180.0 $m^2/g$, more preferably from 30.0 $m^2/g$ to 160.0 $m^2/g$, even more preferably from 40.0 $m^2/g$ to 150.0 $m^2/g$, and most preferably from 50.0 $m^2/g$ to 140.0 $m^2/g$, measured using nitrogen and the BET method according to ISO 9277 77.

According to one embodiment, the surface-reacted calcium carbonate comprises particles having a volume median grain diameter $d_{50}$ of from 2.0 to 50.0 $\mu$m, preferably from 2.5 to 25.0 $\mu$m, more preferably from 2.8 to 20.0 $\mu$m, even more preferably from 3.0 to 10.0 $\mu$m, and most preferably from 4.0 to 8.0 $\mu$m. Preferably, the volume median grain diameter is measured with a Malvern Mastersizer 2000 Laser Diffraction System. The method and the instrument are known to the skilled person and are commonly used to determine grain sizes of fillers and pigments.

Additionally or alternatively, the surface-reacted calcium carbonate comprises particles having a top cut particle size ($d_{98}$) of less than or equal to 40.0 $\mu$m. preferably less than or equal to 30.0 $\mu$m, more preferably less than or equal to 20.0 $\mu$m, still more preferably of less than or equal to 17.0 $\mu$m, more preferably of less than or equal to 14.0 $\mu$m. Preferably, the surface-reacted calcium carbonate comprises particles having a top cut particle size ($d_{98}$) in the range of from 5.0 to 40 $\mu$m, preferably form 6 to 30 $\mu$m, more preferably form 7.0 to 20.0 $\mu$m, still more preferably of from 8.0 to 17.0 $\mu$m, more preferably of from 11.0 to 14.0 $\mu$m.

Preferably, the surface-reacted calcium carbonate has an intra-particle intruded specific pore volume within the range of 0.15 to 1.35 $cm^3/g$, preferably of 0.30 to 1.30 $cm^3/g$, and most preferably of 0.40 to 1.25 $cm^3/g$, calculated from mercury intrusion porosimetry measurement as described in the experimental section. The total pore volume seen in the cumulative intrusion data can be separated into two regions with the intrusion data from 214 $\mu$m down to about 1 - 4 $\mu$m showing the coarse packing of the sample between any agglomerate structures contributing strongly. Below these diameters lies the fine inter-particle packing of the particles themselves. If they also have intra-particle pores, then this region appears bimodal. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired specific pore volume of the internal pores alone as the specific pore volume per unit mass. The same principle of subtraction, of course, applies for isolating any of the other pore size regions of interest.

The pore diameter of the surface-reacted calcium carbonate preferably is in a range of from 4 to 500 nm, more preferably in a range of between 20 and 80 nm, especially from 30 to 70 nm, e.g. 50 nm determined by mercury porosimetry measurement.

According to a preferred embodiment the intra- and/or inter particle pores of the surface-reacted calcium carbonate are hollow and, therefore, the surface-reacted calcium carbonate is unloaded. In other words, the surface-reacted calcium carbonate is not used as a carrying agent.

Preferably, the surface-reacted calcium carbonate has inter-particle voids within the range of from 50 vol.% (v/v) to 99 vol.% (v/v), preferably of from 70 vol.% (v/v) to 98 vol.% (v/v), especially of from 80 vol.% (v/v) to 95 vol.% (v/v) calculated from a mercury porosimetry measurement as described in the experimental section.

Additionally or alternatively, the surface-reacted calcium carbonate has core voids within the range of from 1 vol.% (v/v) to 30 vol.% (v/v), preferably of from 5 vol.% (v/v) to 20 vol.% (v/v), especially of from 10 vol.% (v/v) to 15 vol.% (v/v) calculated from a mercury porosimetry measurement as described in the experimental section.

The surface-reacted calcium carbonate may be in the form of dust or powder and preferably in the form of powder.

According to one embodiment the surface-reacted calcium carbonate does not contain nano-scale particles, e.g. particles with at least one dimension less than 200 nm. A "nano-scale particle" in the meaning of the present invention refers to fine particles with at least one dimension less than 200 nm. According to one embodiment of the present invention, the surface-reacted calcium carbonate does not contain particles that have a number based median particle size $d_{50}$ of less than 200 nm. For determining the number based median particle size $d_{50}$ value, a Malvern Zetasizer Nano ZS can be used employing dynamic light scattering to determine the equivalent spherical hydrodynamic Stokes diameter.

According to step b) of the instant method, at least one pharmaceutically active agent or pharmaceutically inactive precursor thereof is provided.

The expression "at least one" pharmaceutically active agent or pharmaceutically inactive precursor thereof means that one or more pharmaceutically active agent or pharmaceutically inactive precursor thereof may be provided in method step b).

According to one embodiment of the present invention, only one pharmaceutically active agent or pharmaceutically inactive precursor thereof is provided in method step b). According to another embodiment of the present invention, a mixture of two or more pharmaceutically active agents or pharmaceutically inactive precursors thereof is provided in method step b). For example, a mixture of two or three pharmaceutically active agents or pharmaceutically inactive precursors thereof is provided in method step b).

Preferably, only one pharmaceutically active agent or pharmaceutically inactive precursor thereof is provided in method step b).

The at least one pharmaceutically active agent or pharmaceutically inactive precursor thereof is preferably selected from the group comprising pharmaceutically active agent or pharmaceutically inactive precursor of synthetic origin, semi-synthetic origin, natural origin and combinations thereof.

Thus, a pharmaceutically active agent refers to pharmaceutically active agents which are of synthetic origin, semi-synthetic origin, natural origin and combinations thereof. Further, a pharmaceutically inactive precursor of the pharmaceutically active agent refers to pharmaceutically inactive precursors which are of synthetic origin, semi-synthetic origin, natural origin and combinations thereof and will be activated at a later stage to the respective pharmaceutically active agent.

The activation of such pharmaceutically inactive precursor is known to the skilled person and commonly in use, e.g. activation in the stomach and/or gastro-intestinal pathway- such as acidic activation or tryptic- or chimotryptic cleavage.

It lies within the understanding of the skilled person that the mentioned activation methods are of mere illustrative character and are not intended to be of limiting character.

It is to be noted that the at least one pharmaceutically active agent or pharmaceutically inactive precursor thereof, may be any such compound known to the skilled person.

Pharmaceutically active agents thus include any compound that provides prophylactic and/or therapeutic properties when administered to humans and/or animals. Examples include, but are not limited to, pharmaceutical actives, therapeutic actives, veterinarial actives, nutraceuticals, and growth regulators.

For example, the at least one pharmaceutically active agent or pharmaceutically inactive precursor thereof is an anti-tartar agent. Anti-tartar agents useful herein include phosphates. Phosphates include pyrophosphates, polyphosphates, polyphosphonates and mixtures thereof. Pyrophosphates are among the best known phosphates for use in dental care products. Pyrophosphate ions delivered to the teeth derive from pyrophosphate salts. The pyrophosphate salts useful in the present pharmaceutical delivery system include the dialkali metal pyrophosphate salts, tetraalkali metal pyrophosphate salts, and mixtures thereof. Disodium dihydrogen pyrophosphate ($Na_2H_2P_2O_7$), tetrasodium pyrophosphate ($Na_4P_2O_7$), and tetrapotassium pyrophosphate ($K_4P_2O_7$) in their non-hydrated as well as hydrated forms are preferred. Anticalculus phosphates include potassium and sodium pyrophosphates; sodium tripolyphosphate; diphosphonates, such as ethane-1-hydroxy-1,1-diphosphonate; 1 -azacycloheptane- 1,1 - diphosphonate; and linear alkyl diphosphonates; linear carboxylic acids and sodium and zinc citrate.

Agents that may be used in place of or in combination with the above pyrophosphate salt include materials such as synthetic anionic polymers including polyacrylates and copolymers of maleic anhydride or acid and methyl vinyl ether, e.g. Gantrez, as described, for example, in U. S. Patent Number 4,627,977, to Gaffar et al. herein incorporated by reference in its entirety as to the description of such agents, as well as e.g. polyamino propane sulphonic acid (AMPS), zinc citrate trihydrate, polyphosphates, e.g. tripolyphosphate and hexametaphosphate, diphosphonates, e.g. EHDP and AMP, polypeptides, such as polyaspartic and polyglutamic acids, and mixtures thereof.

Antimicrobial agents can also be present in the pharmaceutical delivery system of the present invention as oral agents and/or systemic actives. Such agents may include, but are not urn- ited to, 5-chloro-2-(2, 4-dichlorophenoxy)-phenol, commonly referred to as triclosan, chiorhexidine, alexidine, hexetidine, sanguinarine, benzalkonium chloride, salicylamide, domiphen bromide, cetylpyridiurn chloride (CPC), tetradecyl pyridiniurn chloride (TPC); N-tetradecyl-4-ethyl pyridinium chloride (TDEPC); octenidine; delmopinol, octapinol, and other piperidino derivatives, niacin preparations; zinc/stannous ion agents; antibiotics such as AUGMENTIN, amoxycillin, tetracycline, doxycyline, minocycline, and metronidazole; and analogues, derivatives and salts of the above antimicrobial agents and mixtures thereof.

Anti-inflammatory agents can also be present in the pharmaceutical delivery system of the present invention as oral agents and/or systemic actives. Such agents may include, but are not limited to, non- steroidal anti- inflammatory agents or NSAIDs, such as propionic acid derivatives; acetic acid derivatives; fenamic acid derivatives; biphenylcarboxylic acid derivatives; and oxicams. All of these NSAIDs are fully described in U.S. Patent Number 4,985,459 to Sunshine et al., incorporated by reference herein in its entirety as to the description of such NSAIDs. Examples of useful NSAIDs include acetylsalicylic acid, ibuprofen, naproxen, benoxaprofen, flurbiprofen, fenoprofen, fenbufen, ketoprofen, indoprofen, pirprofen, carprofen, oxaprozin, pranoprofen, microprofen, tioxaprofen, suprofen, alminoprofen, tiaprofenic acid, fluprofen, bucloxic acid and mixtures thereof.

Also useful are the steroidal anti-inflammatory drugs such as hydrocortisone and the like, and COX-2 inhibitors such as meloxicam, celecoxib, rofecoxib, valdecoxib, etoricoxib or mixtures thereof. Mixtures of any of the above anti-inflammatories may be used.

Other materials that can be used in the present pharmaceutical delivery system include commonly known mouth and throat products. These products include, but are not limited to, upper respiratory agents such as phenylephrine, diphenhydramine, dextromethorphan, bromhexine and chiorpheniramine, gastrointestinal agents such as famotidine, loperamide and simethicone, anti-fungals such as miconazole nitrate, antibiotics and analgesics such as ketoprofen and fluribuprofen.

The at least one pharmaceutically active agent or pharmaceutically inactive precursor thereof may be also selected from vitamin E, i.e. tocopheroles, vitamin C, i.e. ascorbic acid and its salts, sodium pyrosulphite, butylhydroxytoluene, butylated hydroxyanisole; and preservatives including parabenes, benzalkonium chloride, chlorbutanol, benzyl alcohol, beta-phenylethyl alcohol, cetylpyridinium chloride, citric acid, tartaric acid, lactic acid, malic acid, acetic acid, benzoic acid, and sorbic acid and their salts; and chelating agents, such as EDTA; and gallates, such as propyl gallate.

The at least one pharmaceutically active agent or pharmaceutically inactive precursor thereof may be also selected from vitamins, such as vitamins B. C and E; minerals, such as fluorides, especially sodium fluoride, sodium monofluoro phosphate and stannous fluoride; - anti- odours, such as zinc and cyclodextrins; propellants, such as 1,1, 2,2-tetrafluoroethane (HFC-134a), optionally being liquefied, and 1,1,1,2, 3,3, 3-heptafluororpropane (HFC-227), optionally being liquefied.

The at least one pharmaceutically active agent or pharmaceutically inactive precursor thereof may be also selected ephedrine, magaldrate, pseudoephedrine, sildenafil, xylocaine, benzalconium chloride, caffeine, phenylephrine, amfepramone, orlistat, sibutramine, acetaminophen, aspirin, aluminium amino acetate, aluminium amino acetate in combination with magnesium oxide, aluminium oxide hydrate in combination with magnesium oxide, calcium carbonate in combination with magnesium hydroxide, calcium carbonate, dihydroxy aluminium sodium carbonate, magnesium oxide, glitazones, metformin, chlorpromazine, dimenhydrinat, domperidone, meclozine, metoclopramide, odansetron, prednisolone, promethazine, acrivastine, cetirizine, cinnarizine, clemastine, cyclizine, desloratadine, dexchlorpheniramine, dimenhydrinate, ebastine, fexofenadine, ibuprofen, levolevoprorion, loratadine, meclozine, mizolastine, promethazine, miconazole, vitamin B12, folic acid, ferro compounds, vitamin C, chlorhexidine diacetate, fluoride, decapeptide KSL, aluminium fluoride, aminochelated calcium, ammonium fluoride, ammonium fluorosilicate, ammonium monofluorphosphate, calcium fluoride, calcium gluconate, calcium glycerophosphate, calcium lactate, calcium monofluorphosphate, calciumcarbonate, carbamide, cetyl pyridinium chloride, chlorhexidine, chlorhexidine digluconate, chlorhexidine chloride, chlorhexidine diacetate, CPP caseine phospho peptide, hexetedine, octadecentyl ammonium fluoride, potassium fluorosilicate, potassium Chloride, potassium monofluorphosphate, sodium bi carbonate, sodium carbonate, sodium fluoride, sodium fluorosilicate, sodium monofluorphosphate, sodium tri polyphosphate, stannous fluoride, stearyl trihydroxyethyl propylenediamine dihydrofluoride, strontium chloride, tetra potassium pyrophosphate, tetra sodium pyrophosphate, tripotassium orthophosphate, trisodium orthophosphate, alginic acid, aluminium hydroxide, sodium bicarbonate, sildenafil, tadalafil, vardenafil, yohimbine, cimetidine, nizatidine, ranitidine, acetylsalicylic acid, clopidogrel, acetylcysteine, bromhexine, codeine, dextromethorphan, diphenhydramine, noscapine, phenylpropanolamine, vitamin D, simvastatin, bisacodyl, lactitol, lactulose, magnesium oxide, sodium picosulfate, senna glycosides, benzocaine, lidocaine, tetracaine, almotriptan, eletriptan, naratriptan, rizatriptan, sumatriptan, zolmitriptan, calcium, chromium, copper, iodine, iron, magnesium, manganese, molybdenium, phosphor, selenium, zinc, chloramine, hydrogenperoxide, metronidazole, triamcinolonacetonide, benzethonium chl., cetyl pyrid. chl., chlorhexidine, fluoride, lidocaine, amphotericin, miconazole, nystatin, fish oil, ginkgo biloba, ginseng, ginger, purple coneflower, saw palmetto, cetirizine, levocetirizine, loratadine, diclofenac, flurbiprofen, acrivastine pseudoephedrine, loratadine pseudoephedrine, glucosamine, hyaluronic acid, decapeptide KSL-W, decapeptide KSL, resveratrol, misoprostol, bupropion, ondansetron HCl, esomeprazole, lansoprazole, omeprazole, pantoprazole, rabeprazole, bacteria and the like, loperamide, simethicone, acetylsalicylic acid and others, sucralfate, vitamin A, vitamin B1, vitamin B 12, vitamin B2, vitamin B6, biotin, vitamin C, vitamin D, vitamin E, folinic acid, vitamin K, niacin, Q10, clotrimazole, fluconazole, itraconazole, ketoconazole, terbinafine, allopurinol, probenecid, atorvastatin, fluvastatin, lovastatin, nicotinic acid, pravastatin, rosuvastatin, simvastatin, pilocarpine, naproxen, alendronate, etidronate, raloxifene, risedronate, benzodiazepines, disulfiram, naltrexone, buprenorphine, codeine, dextropropoxyphene, fentanyl, hydromorphone, ketobemidone, ketoprofen, methadone, morphine, naproxen, nicomorphine, oxycodone, pethidine, tramadol, amoxicillin, ampicillin, azithromycin, ciprofloxacin, clarithromycin, doxycyclin, erythromycin, fusidic acid, lymecycline, metronidazole, moxifloxacin, ofloxacin, oxytetracycline, phenoxymethylpenicillin, rifamycins, roxithromycin, sulfamethizole, tetracycline, trimethoprim, vancomycin, acarbose, glibenclamide, gliclazide, glimepiride, glipizide, insulin, repaglinide, tolbutamide, oseltamivir, aciclovir, famciclovir, penciclovir, valganciclovir, amlopidine, diltiazem, felodipine, nifedipine, verapamil, finasteride, minoxidil, cocaine, buphrenorphin, clonidine, methadone, naltrexone, calcium antagonists, clonidine, ergotamine, β-blockers, aceclofenac, celecoxib, dexiprofen, etodolac, indometacin, ketoprofen, ketorolac, lornoxicam, meloxicam, nabumetone, oiroxicam, parecoxib, phenylbutazone, piroxicam, tiaprofenic acid, tolfenamic acid, aripiprazole, chlorpromazine, chlorprothixene, clozapine, flupentixol, fluphenazine, haloperidol, lithium carbonate, lithium citrate, melperone, penfluridol, periciazine, perphenazine, pimozide, pipamperone, prochlorperazine, risperidone, thioridizin, fluconazole, itraconazole, ketoconazole, voriconazole, opium, benzodiazepines, hydroxine, meprobamate, phenothiazine, aluminiumaminoacetate, esomeprazole, famotidine, magnesium oxide, nizatide, omeprazole, pantoprazole, fluconazole, itraconazole, ketoconazole, metronidazole, amphetamine, atenolol, bisoprolol fumarate, metoprolol, metropolol, pindolol, propranolol, auranofin, and bendazac.

Further examples of useful at least one pharmaceutically active agents or pharmaceutically inactive precursors thereof include active ingredients selected from the therapeutical groups comprising: Analgesic, Anaestetic, Antipyretic, Anti allergic, Anti-arrhythmic, Appetite suppressant, Antifungal, Anti-inflammatory, Broncho dilator, Cardiovascular drugs, Coronary dilator, Cerebral dilator, Peripheral vasodilator, Anti-infective, Psychotropic, Anti- manic, Stimulant, Antihistamine, Laxative, Decongestrant, Gastro-intestinal sedative, Sexual dysfunction agent, Desinfectants, Anti-diarrheal, Anti-anginal substance, Vasodilator, Anti-hypertensive agent, Vasoconstrictor, Migraine treating agent, Antibiotic, Tranquilizer, Antipsychotic, Anti-tumour drug, Anticoagulant, Antithrombotic agent, Hypnotic, Sedative, Anti-emetic, Anti-, nauseant, Anticonvulsant, Neuromuscular agent, Hyper and hypoglycaemic, Thyroid and antithyroid, Diuretic, Antispasmodic, Uterine relaxant, Anti-obesity agent, Anoretic, Spasnolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretc, Anti-flatulent, Betablokker, Teeth Whitener, Enzyme, Co-enzyme, Protein, Energy booster, Fibre, Probiotics, Prebiotics, Antimicrobial agent, NSAID, Anti-tussives, Decongestrants, Anti-histamines, Expectorants, Anti-diarrheals, Hydrogen antagonists, Proton pump inhibitors, General nonselective CNS depressants, General nonselective CNS stimulants, Selectively CNS function modyfing drugs, Antiparkinsonism, Narcotic-analgetics, Analgetic-antipyretics, Psychopharmacological drugs, and Sexual dysfunction agents.

Examples of useful at least one pharmaceutically active agents or pharmaceutically inactive precursors thereof may also include: Casein glyco-macro-peptide (CGMP), Triclosan, Cetyl pyridinium chloride, Domiphen bromide, Quarternary ammonium salts, zinc components, Sanguinarine, Fluorides, Alexidine, Octonidine, EDTA, Aspirin, Acetaminophen, Ibuprofen, Ketoprofen, Diflunisal, Fenoprofen calcium, Naproxen, Tolmetin sodium, Indomethacin, Benzonatate, Caramiphen edisylate, Menthol, Dextromethorphan hydrobromide, Theobromine hydrochloride, Chlophendianol Hydrochloride, Pseudoephedrine Hydrochloride, Phenylephrine, Phenylpropanolamine, Pseudoephedrine sulphate, Brompheniramine maleate, Chlorpheniramine- maleate, Carbinoxamine maleate, Clemastine fumarate, Dexchlorpheniramine maleate, Dephenhydramine hydrochloride, Diphenpyralide hydrochloride, Azatadine maleate, Diphenhydramine citrate, Doxylamine succinate, Promethazine hydrochloride, Pyrilamine maleate, Tripellenamine citrate, Triprolidine hydrochloride, Acrivastine, Loratadine, Brompheniramine, Dexbrompheniamine, Guaifenesin, Ipecac, potassium iodide, Terpin hydrate, Loperamide, Famotidine, Ranitidine, Omeprazole, Lansoprazole, Aliphatic alcohols, Barbiturates, caffeine, strychnine, Picrotoxin, Pentyenetetrazol, Phenyhydantoin, Phenobarbital, Primidone, Carbamazapine, Etoxsuximide, Methsuximide, Phensuximide, Trimethadione, Diazepam, Benzodiazepines, Phenacemide, Pheneturide, Acetazolamide, Sulthiame, bromide, Levodopa, Amantadine, Morphine, Heroin, Hydromorphone, Metopon, Oxymorphone, Levophanol, Codeine, Hydrocodone, Xycodone, Nalorphine, Naloxone, Naltrexone, Salicylates, Phenylbutazone, Indomethacin, Phenacetin, Chlorpromazine, Methotrimeprazine, Haloperidol, Clozapine, Reserpine, Imipramine, Tranylcypromine, Phenelzine, Lithium, Sildenafil citrate, Tadalafil, and Vardenafil CL.

Examples of useful at least one pharmaceutically active agents or pharmaceutically inactive precursors thereof may include active ingredients selected from the groups of ace-inhibitors, antianginal drugs, anti- arrhythmias, anti-asthmatics, anti-cholesterolemics, analgesics, anesthetics, anticonvulsants, anti-depressants, antidiabetic agents, anti-diarrhea preparations, antidotes, anti-histamines, anti-hypertensive drugs, anti-inflammatory agents, anti-lipid agents, anti- manics, antinauseants, anti-stroke agents, anti-thyroid preparations, anti-tumour drugs, anti- viral agents, acne drugs, alkaloids, amino acid preparations, anti-tussives, anti- uricemic drugs, anti- viral drugs, anabolic preparations, systemic and non-systemic anti-infective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocriptine, sympatholytics, tremor preparations, urinary tract agents, vasodilators, laxatives, antacids, ion exchange resins, anti-pyretics, appetite suppressants, expectorants, anti-anxiety agents, antiulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumour drugs, anti-coagulants, anti-thrombotic drugs, hypnotics, anti-emetics, antinauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of useful at least one pharmaceutically active agents or pharmaceutically inactive precursors thereof contemplated for use in the present pharmaceutical delivery system can include antacids, H2-antagonists, and analgesics. For example, antacid dosages can be prepared using the ingredients calcium carbonate alone or in combination with magnesium hydroxide, and/or aluminium hydroxide. Moreover, antacids can be used in combination with H2-antagonists.

Analgesics include opiates and opiate derivatives, such as Oxycontin™, ibuprofen, aspirin, acetaminophen, and combinations thereof that may optionally include caffeine.

Other useful at least one pharmaceutically active agents or pharmaceutically inactive precursors thereof for use in embodiments can include anti-diarrheals such as Immodium™ AD, anti-histamines, anti-tussives, decongestants, vitamins, and breath fresheners. Also contemplated for use herein are anxiolytics such as Xanax™; anti-psychotics such as Clozaril™ and Haldol™; non-steroidal anti-inflammatories (NSAID's) such as ibuprofen, naproxen sodium, Voltaren™ and Lodine™, anti-histamines such as Claritin™, Hismanal™, Relafen™, and Tavist™; antiemetics such as Kytril™ and Cesamet™; bronchodilators such as Bentolin™, Proventil™; anti-depressants such as Prozac™, Zoloft™, and Paxil™; anti-migraines such as Imigra™, ACE-inhibitors such as Vasotec™, Capoten™ and Zestril™; anti-Alzheimer's agents, such as Nicergoline™; and CaH-antagonists such as Procardia™, Adalat™, and Calan™.

The popular H2-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients can include, but are not limited to, the following: aluminium hydroxide, dihydroxyaluminium aminoacetate, aminoacetic acid, aluminium phosphate, dihydroxyaluminium sodium carbonate, bicarbonate, bismuth aluminate, bismuth carbonate, bismuth subcarbonate, bismuth subgallate, bismuth subnitrate, bismuth subsilysilate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminium mono-ordibasic calcium phosphate, tricalcium phosphate, potassium bicarbonate, sodium tartrate, sodium bicarbonate, magnesium aluminosilicates, tartaric acids and salts. A variety of nutritional supplements may also be used as the at least one pharmaceutically active agent or pharmaceutically inactive precursor thereof including virtually any vitamin or mineral. For example, vitamin A, vitamin C, vitamin D, vitamin E, vitamin K, vitamin B6, vitamin B 12, thiamine, riboflavin, biotin, folic acid, niacin, pantothenic acid, sodium, potassium, calcium, magnesium, phosphorus, sulfur, chlorine, iron, copper, iodine, zinc, selenium, manganese, choline, chromium, molybdenum, fluorine, cobalt and combinations thereof, may be used. Examples of nutritional supplements that can be used as active ingredients are set forth in U.S. Patent Application Publication Nos. 2003/0157213 A1, 2003/0206993 and 2003/0099741 A1 which are incorporated in their entirety herein by reference for all purposes. Various herbals may also be used as the at least one pharmaceutically active agent or pharmaceutically inactive precursor thereof such as those with various medicinal or dietary supplement properties. Herbals are generally aromatic plants or plant parts and or extracts thereof that can be used medicinally or for flavoring. Suitable herbals can be used singly or in various mixtures. Commonly used herbs include Echinacea, Goldenseal, Calendula, Rosemary, Thyme, Kava Kava, Aloe, Blood Root, Grapefruit Seed Extract, Black Cohosh, Ginseng, Guarana, Cranberry, Ginko Biloba, St. John's Wort, Evening Primrose Oil, Yohimbe Bark, Green Tea, Ma Huang, Maca, Bilberry, Lutein, and combinations thereof.

In some embodiments, minerals can be include but are not limited to sodium, magnesium, chromium, iodine, iron, manganese, copper, fluoride, potassium, phosphorous, molybdenum, selenium, zinc, and combinations thereof.

In some embodiments the at least one pharmaceutically active agent or pharmaceutically inactive precursor thereof can include but is not limited to L-carnitine, choline, coenzyme Q10, alpha-lipoic acid, omega-3 -fatty acids, pepsin, phytase, trypsin, lipases, proteases, cellulases, and combinations thereof.

The at least one pharmaceutically active agent or pharmaceutically inactive precursor thereof can also include ascorbic acid, citric acid, rosemary oil, vitamin A, vitamin E, vitamin E phosphate, tocopherols, di-alpha- tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In some embodiments, the at least one pharmaceutically active agent or pharmaceutically inactive precursor thereof can be selected from phytochemicals such as cartotenoids, chlorophyll, chlorophyllin, fibre, flavanoids, anthocyanins, cyaniding, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof.

In some embodiments, the at least one pharmaceutically active agent or pharmaceutically inactive precursor thereof can be selected from analgesics/anesthetics such as menthol, phenol, hexylresorcinol, benzocaine, dyclonine hydrochloride, benzyl alcohol, salicyl alcohol, and combinations thereof. In some embodiments, the at least one pharmaceutically active agent or pharmaceutically inactive precursor thereof can be selected from demulcents such as slippery elm bark, pectin, gelatin, and combinations thereof. In some embodiments, the at least one pharmaceutically active agent or pharmaceutically inactive precursor thereof can be selected from antiseptic ingredients such as cetylpyridinium chloride, domiphen bromide, dequalinium chloride, and combinations thereof.

In some embodiments, the at least one pharmaceutically active agent or pharmaceutically inactive precursor thereof can be selected from antitussive ingredients such as chlophedianol hydrochloride, codeine, codeine phosphate, codeine sulfate, dextromethorphan, dextromethorphan hydrobromide, diphenhydramine citrate, and diphenhydramine hydrochloride, and combinations thereof.

In some embodiments, the at least one pharmaceutically active agent or pharmaceutically inactive precursor thereof can be selected from throat soothing agents such as honey, propolis, aloe vera, glycerine, menthol and combinations thereof can be included. In still other embodiments, the at least one pharmaceutically active agent or pharmaceutically inactive precursor thereof can be selected from cough suppressants. Such cough suppressants can fall into two groups: those that alter the texture or production of phlegm such as mucolytics and expectorants; and those that suppress the coughing reflex such as codeine (narcotic cough suppressants), antihistamines, dextromethorphan and isoproterenol (non-narcotic cough suppressants). In some embodiments, ingredients from either or both groups can be included.

In still other embodiments, the at least one pharmaceutically active agent or pharmaceutically inactive precursor thereof can be an antitussive selected from the group comprising codeine, dextromethorphan, dextrorphan, diphenhydramine, hydrocodone, noscapine, oxycodone, pentoxyverine and combinations thereof. In some embodiments, the at least one pharmaceutically active agent or pharmaceutically inactive precursor thereof can be selected from antihistamines such as acrivastine, azatadine, brompheniramine, chlo[phi]heniramine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof. In some embodiments, the at least one pharmaceutically active agent or pharmaceutically inactive precursor thereof can be selected from non-sedating antihistamines such as astemizole, cetirizine, ebastine, fexofenadine, loratidine, terfenadine, and combinations thereof.

In some embodiments, the at least one pharmaceutically active agent or pharmaceutically inactive precursor thereof can be selected from expectorants as ammonium chloride, guaifenesin, ipecac fluid extract, potassium iodide and combinations thereof. In some embodiments, the at least one pharmaceutically active agent or pharmaceutically inactive precursor thereof can be selected from mucolytics such as acetylcycsteine, ambroxol, bromhexine and combinations thereof. In some embodiments, the at least one pharmaceutically active agent or pharmaceutically inactive precursor thereof can be selected from analgesic, antipyretic and anti-inflammatory agents such as acetaminophen, aspirin, diclofenac, diflunisal, etodolac, fenoprofen, flurbiprofen, ibuprofen, ketoprofen, ketorolac, nabumetone, naproxen, piroxicam, caffeine and mixtures thereof. In some embodiments, the at least one pharmaceutically active agent or pharmaceutically inactive precursor thereof can be selected from local anesthetics such as lidocaine, benzocaine, phenol, dyclonine, benzonotate and mixtures thereof. In some embodiments, the at least one pharmaceutically active agent or pharmaceutically inactive precursor thereof can be selected from nasal decongestants and ingredients that provide the perception of nasal clearing. In some embodiments, such as nasal decongestants can include but are not limited to phenylpropanolamine, pseudoephedrine, ephedrine, phenylephrine, oxymetazoline, and combinations thereof. In some embodiments, the at least one pharmaceutically active agent or pharmaceutically inactive precursor thereof can be selected from ingredients that provide a perception of nasal clearing such as menthol, camphor, borneol, ephedrine, eucalyptus oil, peppermint oil, methyl salicylate, bornyl acetate, lavender oil, wasabi extracts, horseradish extracts, and combinations thereof. In some embodiments, a perception of nasal clearing can be provided by odoriferous essential oils, extracts from woods, gums, flowers and other botanicals, resins, animal secretions, and synthetic aromatic materials.

For example, the at least one pharmaceutically active agent or pharmaceutically inactive precursor thereof is paracetamol.

It is appreciated that the pharmaceutical delivery system may further comprise additives which are typically used in such systems, such as colouring agents, flavours, disintegrants such as croscarmellose sodium, crospovidone or sodium starch glycolate, fumed silica such as Aerosil®, e.g. Aerosil ® 200 of Evonik Industries AG, Germany, etc.

According to step c) of the instant method, at least one formulating aid is provided.

The expression "at least one" formulating aid means that one or more formulating aid may be provided in method step c).

According to one embodiment of the present invention, only one formulating aid is provided in method step c). According to another embodiment of the present invention, a mixture of two or more formulating aids is provided in method step c). For example, a mixture of two or three formulating aids is provided in method step c).

Preferably, only one formulating aid is provided in method step c).

In one embodiment, the at least one formulating aid is at least one inner-phase lubricant and/or outer-phase lubricant. For example, the at least one formulating aid is at least one inner-phase lubricant or outer-phase lubricant, preferably at least one inner-phase lubricant. Alternatively, the at least one formulating aid is at least one inner-phase lubricant and outer-phase lubricant.

Said at least one inner-phase lubricant can be selected from the group comprising sorbitan esters of fatty acids and polyoxyethylated hydrogenated castor oil (e.g. the product sold under the trade name CREMOPHOR ®), block copolymers of ethylene oxide and propylene oxide (e.g. products sold under trade names PLURONIC ® and POLOXAMER), polyoxyethylene fatty alcohol ethers, polyoxyethylene sorbitan fatty acid esters, sorbitan esters of fatty acids and polyoxyethylene steraric acid esters, stearyl alcohol, glycerol dibehenate, sodium stearyl fumarate, glycerol distearate and combinations thereof. Preferably, said at least one inner-phase lubricant is sodium stearyl fumarate.

Said at least one outer-phase lubricant can be selected from the group comprising lecithin, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllactylate, magnesium and/or calcium stearate, hydrogenated vegetable oils, stearic acid, sodium lauryl sulphate, magnesium lauryl sulphate, colloidal silica, talc and combinations thereof.

Preferably, said at least one outer-phase lubricant is magnesium and/or calcium stearate, more preferably magnesium stearate.

In one embodiment, the at least one formulating aid is provided in a total amount from about 0.1 wt.-% to about 10.0 wt.-%, preferably from about 0.3 wt.-% to about 5.0 wt%, more preferably from about 0.5 wt.-% to about 2.5 wt.-% based on the total weight of the pharmaceutical delivery system.

According to step d) of the instant method, the surface-reacted calcium carbonate of step a), the at least one pharmaceutically active agent or pharmaceutically inactive precursor thereof of step b) and the at least one formulating aid of step c) are mixed.

Mixing the surface-reacted calcium carbonate of step a) with the at least one pharmaceutically active agent or pharmaceutically inactive precursor thereof of step b) and the at least one formulating aid of step c) can be carried out simultaneously or separately in any order to form a mixture.

In one embodiment of the present invention, method step d) is carried out in that the surface-reacted calcium carbonate of step a) is combined simultaneously with the at least one pharmaceutically active agent or pharmaceutically inactive precursor thereof of step b) and the at least one formulating aid of step c). For example, method step d) is carried out in that the surface-reacted calcium carbonate of step a) is combined with a blend consisting of the at least one pharmaceutically active agent or pharmaceutically inactive precursor thereof of step b) and the at least one formulating aid of step c). That is to say, said at least one pharmaceutically active agent or pharmaceutically inactive precursor thereof of step b) and the at least one formulating aid of step c) may be pre-mixed prior to addition to said surface-reacted calcium carbonate of step a).

Preferably, mixing step d) is carried out in that the at least one pharmaceutically active agent or pharmaceutically inactive precursor thereof of step b) and the at least one formulating aid of step c) are added independently from each other to the surface-reacted calcium carbonate of step a).

For the purposes of the present invention, any suitable mixing means known in the art may be used. However, mixing step d) preferably takes place in a mixer and/or blender, preferably a mixer such as a tumbling mixer.

It is preferred that the mixture obtained in method step d) is free of agglomerates. Thus, the mixture obtained in method step d) is preferably sieved, e.g. through a 1 000 μm sieve, before method step e) is carried out.

According to step e) of the instant method, the mixture obtained in step d) is compacted by means of a roller compacter at a compaction pressure in the range from 4 to 20 bar.

The term "roller compacting" refers to a process in which fine powders are forced between two counter rotating rolls and pressed into a solid compact or ribbon.

For the purposes of the present invention, roller compacting can be carried out with any suitable roller compactor known to the skilled person. For example, roller compacting is carried out with a Fitzpatrick® Chilsonator IR220 roller compactor of the Fitzpatrick Company, USA.

It is one requirement of the instant method that method step e) is carried out at a compaction pressure in the range from 4 to 20 bar. Preferably, roller compacting step e) is carried out at a roller compaction pressure in the range from 4 to 15 bar, more preferably in the range from 4 to 10 bar and most preferably in the range from 4 to 7 bar.

Additionally or alternatively, the feed rate and/or the roll speed during roller compacting step e) is/are adjusted such that a ribbon thickness of from 0.4 to 0.8 mm, preferably from 0.5 to 0.7 mm and most preferably of about 0.6 mm is obtained. For example, the feed rate or the roll speed during roller compacting step e) is adjusted such that a ribbon thickness of from 0.4 to 0.8 mm, preferably from 0.5 to 0.7 mm and most preferably of about 0.6 mm is obtained. Alternatively, the feed rate and the roll speed during roller compacting step e) are adjusted such that a ribbon thickness of from 0.4 to 0.8 mm, preferably from 0.5 to 0.7 mm and most preferably of about 0.6 mm is obtained.

According to step f) of the instant method, the roller compacted mixture obtained in step e) is compacted for obtaining the pharmaceutical delivery system.

Compacting can be carried out with any conventional compactor known to the skilled person. For example, compacting is carried out with a tablet press such as a Styl'One 105 ml tablet press from Medel'Pharm, France.

Preferably, compacting step f) is a pelletizing or tableting step.

The term "pelletizing" in the meaning of the present invention refers to a process of compacting or moulding a material into the shape of a pellet. The term "tableting" in the meaning of the present invention refers to a process of compacting or moulding a material into the shape of a tablet.

It is preferred that method step f) is carried out at a compressive pressure in the range from 5 to 500 MPa. It is to be noted that the compressive pressure used in step f) depends on the specific at least one pharmaceutically active agent or pharmaceutically inactive precursor thereof provided in step b). The skilled person will thus adapt the compressive pressure accordingly. Preferably, compacting step f) is carried out at a compressive pressure in the range from 6 to 400 MPa, and most preferably in the range from 10 to 400 MPa. For example, compacting step f) is carried out at a compressive pressure in the range from 50 to 300 MPa, and most preferably in the range from 50 to 200 MPa or from 100 to 200 MPa.

In one embodiment, the roller compacted mixture obtained in roller compacting step e) is subjected to a milling step before compacting step f) is carried out.

Milling can be carried out with any conventional mill known to the skilled person. For example, milling is carried out with a FitzMill® from the Fitzpatrick Company, USA.

Additionally or alternatively, the roller compacted mixture obtained in roller compacting step e) is subjected to a sieving step before compacting step f) is carried out.

If the method comprises a milling and sieving of the mixture obtained in roller compacting step e), said mixture is preferably first subjected to the milling step followed by the sieving step, before compacting step f) is carried out. That is to say, the material obtained by sieving is subjected to compacting step f).

Sieving can be carried out with any conventional sieving means known to the skilled person. For example, sieving is carried out with a Vibrating sieve tower of Vibro Retsch, Switzerland.

In order to receive a robust pharmaceutical delivery system, the roller compacted mixture subjected to compacting step f) preferably has a specific stability. Said stability is preferably determined in a stability test.

The stability of the roller compacted mixture subjected to compacting step f) is preferably determined by the remaining fraction of the roller compacted mixture. Preferably, the roller compacted mixture subjected to compacting step f) has a remaining fraction of at least 60 wt-% after sieving the roller compacted mixture on a sieve having 500 $\mu$m mesh size, the remaining fraction is calculated by Formula (III)

$$RF\,\text{wt.} - \% = 100 - \left( \frac{(m_{\text{tot}} - m_{\text{res}}) \times 100}{m_{\text{tot}}} \right) \qquad \text{(III)}$$

wherein $m_{\text{tot}}$ is the total mass applied initially on the sieve (g) and $m_{\text{res}}$ is the residual amount on the sieve (g) after 10 minutes of shaking.

In one embodiment of the present invention, advantageous pharmaceutical delivery systems are obtained if the roller compacted mixture subjected to compacting step f) has a specific grain size distribution. Thus, it is preferred that the roller compacted mixture subjected to compacting step f) has a grain size of from 180 to 710 $\mu$m obtained by sieving on different mesh sizes, preferably by sieving with mesh sizes in the order of 180 $\mu$m, 250 $\mu$m, 355 $\mu$m, 500 $\mu$m and 710 $\mu$m More preferably, by sieving with mesh sizes in the order of 180 $\mu$m, 250 $\mu$m, 355 $\mu$m, 500 $\mu$m and 710 $\mu$m and combining the sieved roller compacted mixture such that grain sizes of less than 180 $\mu$m and more than 710 $\mu$m are excluded.

Thus, the method for the production of a pharmaceutical delivery system preferably comprises that the stable roller compacted mixture having a remaining fraction of at least 60 % after sieving the roller compacted mixture on a sieve having 500 $\mu$m mesh size as described above is subjected to a milling and sieving such that the obtained roller compacted mixture has a grain size of from 180 to 710 $\mu$m obtained by sieving on different mesh sizes. In this case, the roller compacted mixture obtained in step e) is preferably first subjected to the stability test as described above, followed by a milling and sieving of the stable roller compacted mixture such that the roller compacted mixture has a grain size of from 180 to 710 $\mu$m obtained by sieving on different mesh sizes, before compacting step f) is carried out. That is to say, the material obtained by sieving such that the roller compacted mixture has a grain size of from 180 to 710 $\mu$m obtained by sieving on different mesh sizes is subjected to compacting step f).

The resulting pharmaceutical delivery system may be a tablet, mini-tablet, capsule or pellet.

The pharmaceutical delivery system thus may be prepared in a wide size range, wherein different size fractions may be separated by conventional means such as sieving.

Generally, the pharmaceutical delivery system may have a weight median particle size of from 0.1 to 20.0 mm, preferably 0.2 to 15.0 mm and more preferably from 0.3 to 10.0 mm.

The pharmaceutical delivery system obtained by the process according to the present invention has turned out to have a low friability. Preferably, the pharmaceutical delivery system has a friability of $\leq$ 1.10 %, measured according to Ph.Eur.4 on tablets compressed at 100 MPa. More preferably, the pharmaceutical delivery system has a friability of $\leq$ 1.0 %, even more preferably $\leq$ 0.8 % and most preferably $\leq$ 0.6 %, like from 0.4 to 0.6 % measured according to Ph.Eur.4 on tablets compressed at 100 MPa.

Additionally or alternatively, the pharmaceutical delivery system fulfils Formula (I)

$$(FR_{RCPh}) / (FR_{RCPl}) \geq 1.2 \qquad (I)$$

wherein

$(FR_{RCPh})$ is the friability (in %), measured according to Ph.Eur.4, of a pharmaceutical delivery system RCPh, wherein the compaction pressure in step e) is at least 15 bar;
$(FR_{RCPl})$ is the friability (in %), measured according to Ph.Eur.4, of a pharmaceutical delivery system RCPl, wherein the compaction pressure in step e) is less than the compaction pressure used for obtaining the pharmaceutical delivery system RCPh.

Preferably, the pharmaceutical delivery system fulfils Formula (Ia), more preferably (Ib) and most preferably (Ic)

$$(FR_{RCPh}) / (FR_{RCPl}) \geq 1.5 \qquad (Ia)$$

$$(FR_{RCPh}) / (FR_{RCPl}) \geq 1.7 \qquad (Ib)$$

$$2.5 \geq (FR_{RCPh}) / (FR_{RCPl}) \geq 1.7 \qquad (Ic)$$

wherein

$(FR_{RCPh})$ is the friability (in %), measured according to Ph.Eur.4, of a pharmaceutical delivery system RCPh, wherein the compaction pressure in step e) is at least 15 bar;
$(FR_{RCPl})$ is the friability (in %), measured according to Ph.Eur.4, of a pharmaceutical delivery system RCPl, wherein the compaction pressure in step e) is less than the compaction pressure used for obtaining the pharmaceutical delivery system RCPh.

Additionally or alternatively, the pharmaceutical delivery system obtained by the process according to the present invention has turned out to have a high flowability. Preferably, the pharmaceutical delivery system fulfils Formula (II)

$$(FL_{RCP}) / (FL_{reference}) \geq 1.1 \qquad (II)$$

wherein

$(FL_{RCP})$ is the flowability (in g/s), measured according to Ph.Eur.4 at an opening diameter of 7 mm, of the pharmaceutical delivery system;
$(FL_{reference})$ is the flowability (in g/s), measured according to Ph.Eur.4 at an opening diameter of 7 mm, of the same pharmaceutical delivery system, in which the surface-reacted calcium carbonate is replaced by microcrystalline cellulose.

Preferably, the pharmaceutical delivery system fulfils Formula (IIa), more preferably (IIb) and most preferably (IIc)

$$2.0 \geq (FL_{RCP}) / (FL_{reference}) \geq 1.1 \qquad (IIa)$$

$$1.8 \geq (FL_{RCP}) / (FL_{reference}) \geq 1.1 \qquad (IIb)$$

$$1.5 \geq (FL_{RCP}) / (FL_{reference}) \geq 1.1 \qquad (IIc)$$

wherein

$(FL_{RCP})$ is the flowability (in g/s), measured according to Ph.Eur.4 at an opening diameter of 7 mm, of the pharmaceutical delivery system;

$(FL_{reference})$ is the flowability (in g/s), measured according to Ph.Eur.4 at an opening diameter of 7 mm, of the same pharmaceutical delivery system, in which the surface-reacted calcium carbonate is replaced by microcrystalline cellulose.

In view of the good results obtained the present invention refers in another aspect to the pharmaceutical delivery system, preferably a tablet, mini-tablet, capsule or pellet, obtained by the method.

Another aspect refers to the use of a surface-reacted calcium carbonate for improving the friability of a pharmaceutical delivery system, wherein the surface-reacted calcium carbonate is a reaction product of natural ground or precipitated calcium carbonate with carbon dioxide and one or more acids in an aqueous medium, wherein the carbon dioxide is formed in situ by the acid treatment and/or is supplied from an external source, wherein the pharmaceutical delivery system

a) has a friability of $\leq$ 1.10 %, measured according to Ph.Eur.4 on tablets compressed at 100 MPa; and/or
b) fulfils Formula (II)

$$(FR_{RCPh}) / (FR_{RCPl}) \geq 1.2 \qquad (I)$$

wherein

$(FR_{RCPh})$ is the friability (in %), measured according to Ph.Eur.4, of a pharmaceutical delivery system RCPh, wherein the compaction pressure in step e) is at least 15 bar;
$(FR_{RCPl})$ is the friability (in %), measured according to Ph.Eur.4, of a pharmaceutical delivery system RCPl, wherein the compaction pressure in step e) is less than the compaction pressure used for obtaining the pharmaceutical delivery system RCPh.

With regard to the definition of the surface-reacted calcium carbonate, the pharmaceutical delivery system and preferred embodiments thereof, reference is made to the statements provided above when discussing the technical details of the surface-reacted calcium carbonate and the pharmaceutical delivery system of the present invention.

In one embodiment, the pharmaceutical delivery system has a friability of $\leq$ 1.0 %, even more preferably $\leq$ 0.8 % and most preferably $\leq$ 0.6 %, like from 0.4 to 0.6 % measured according to Ph.Eur.4 on tablets compressed at 100 MPa.

Additionally or alternatively, the pharmaceutical delivery system fulfils Formula (Ia), more preferably (Ib) and most preferably (Ic)

$$(FR_{RCPh}) / (FR_{RCPl}) \geq 1.5 \qquad (Ia)$$

$$(FR_{RCPh}) / (FR_{RCPl}) \geq 1.7 \qquad (Ib)$$

$$2.5 \geq (FR_{RCPh}) / (FR_{RCPl}) \geq 1.7 \qquad (Ic)$$

wherein

$(FR_{RCPh})$ is the friability (in %), measured according to Ph.Eur.4, of a pharmaceutical delivery system RCPh, wherein the compaction pressure in step e) is at least 15 bar;
$(FR_{RCPl})$ is the friability (in %), measured according to Ph.Eur.4, of a pharmaceutical delivery system RCPl, wherein the compaction pressure in step e) is less than the compaction pressure used for obtaining the pharmaceutical delivery system RCPh.

A further aspect of the present invention refers to the use of a surface-reacted calcium carbonate for improving the flowability of a pharmaceutical delivery system, wherein the surface-reacted calcium carbonate is a reaction product of natural ground or precipitated calcium carbonate with carbon dioxide and one or more acids in an aqueous medium, wherein the carbon dioxide is formed in situ by the acid treatment and/or is supplied from an external source, wherein the pharmaceutical delivery system fulfils Formula (II)

$$(FL_{RCP}) \, / \, (FL_{reference}) \geq 1.1 \quad (II)$$

wherein

($FL_{RCP}$) is the flowability (in g/s), measured according to Ph.Eur.4 at an opening diameter of 7 mm, of the pharmaceutical delivery system;
($FL_{reference}$) is the flowability (in g/s), measured according to Ph.Eur.4 at an opening diameter of 7 mm, of the same pharmaceutical delivery system, in which the surface-reacted calcium carbonate is replaced by microcrystalline cellulose

With regard to the definition of the surface-reacted calcium carbonate, the pharmaceutical delivery system and preferred embodiments thereof, reference is made to the statements provided above when discussing the technical details of the surface-reacted calcium carbonate and the pharmaceutical delivery system of the present invention.
In one embodiment of the present invention, the pharmaceutical delivery system fulfils Formula (IIa), more preferably (IIb) and most preferably (IIc)

$$2.0 \geq (FL_{RCP}) \, / \, (FL_{reference}) \geq 1.1 \quad (IIa)$$

$$1.8 \geq (FL_{RCP}) \, / \, (FL_{reference}) \geq 1.1 \quad (IIb)$$

$$1.5 \geq (FL_{RCP}) \, / \, (FL_{reference}) \geq 1.1 \quad (IIc)$$

wherein

($FL_{RCP}$) is the flowability (in g/s), measured according to Ph.Eur.4 at an opening diameter of 7 mm, of the pharmaceutical delivery system;
($FL_{reference}$) is the flowability (in g/s), measured according to Ph.Eur.4 at an opening diameter of 7 mm, of the same pharmaceutical delivery system, in which the surface-reacted calcium carbonate is replaced by microcrystalline cellulose.

**Brief description of the figures**

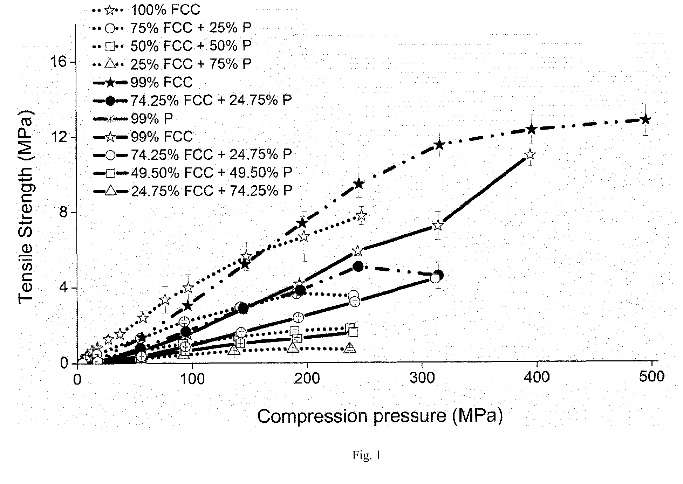

**Fig. 1:** Tensile strength of FCC based tablet formulations as a function of compressive pressure. Tablets contained varying amounts of paracetamol (P) and were prepared using FCC powder (dotted lines) or FCC granules obtained by roller compaction at 5 bar (dashed/dotted line and dark symbol) or 20 bar (black solid line).

All granules contained 1wt.-% sodium stearyl fumarate. Values are means $\pm$ SD of $n$=3 experiments.

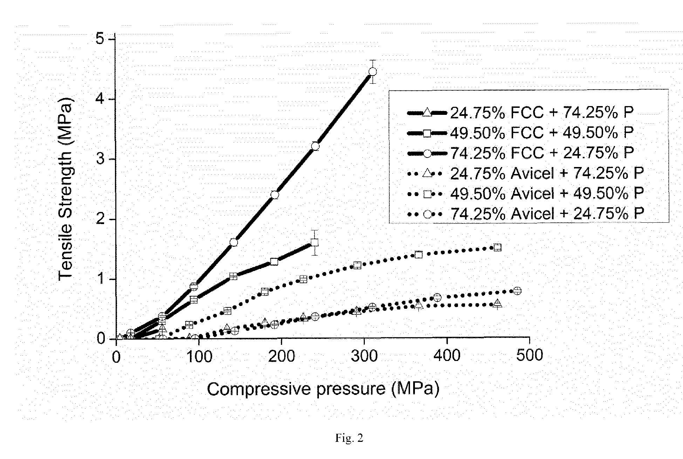

Fig. 2: Compaction of granules containing mixtures of paracetamol (P) and FCC or Avicel. Comparison of tensile strength of tablets as a function of compressive pressure. All formulations were granulated at 20 bar and contained 1% sodium stearyl fumarate. Values are means $\pm$ SD of $n$=3 experiments.

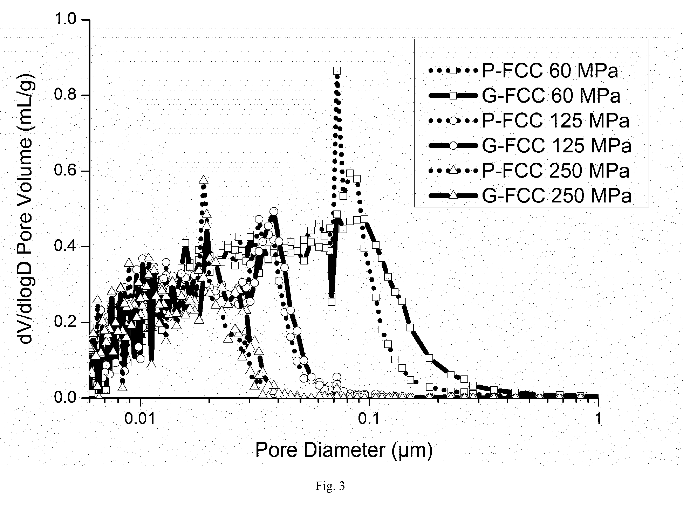

Fig 3: Porosity of FCC after compaction. Comparison of compacted FCC powder (P-FCC) or FCC granules (G-FCC). G-FCC was granulated at 5 bar. Mercury porosimetry was used to analyze the pore size distribution of FCC at increasing compaction pressures. Values are means of $n$=2 measurements.

The following examples and tests will illustrate the present invention, but are not intended to limit the invention in any way.

### Examples

**Materials and methods**

*1.1 Materials*

Functionalized calcium carbonate (FCC), (from Omya International AG, Switzerland) was compared to microcrystalline cellulose (Avicel® PH 102, FMC BioPolymer, Ireland). Further details of the functionalized calcium carbonate are summarized in the following table 1:

Table 1:

| Apparent true density [g/cm$^3$] | Mean weight median particle size [$\mu$m] | Top cut particle size $d_{98}$ [$\mu$m] | BET Specific surface area [m$^2$/g] | Core voids [%, v/v] | Stratum / and interparticle voids [%, v/v] | Intra-particle intruded specific pore volume [cm$^3$/g] |
|---|---|---|---|---|---|---|
| 2.7259 | 6.80 | 13.2 | 56.22 | 11 | 89 | 0.97 |

Paracetamol was chosen as pharmaceutical active agent (Acetaminophen USP / Paracetamol Ph Eur Powder, Mallinckrodt, USA).

Sodium stearyl fumarate (SSF, LubriSanaq®, Pharmatrans Sanaq AG, Switzerland) was used as an inner phase lubricant during roller compaction, whereas magnesium stearate (MgSt, Sandoz, Switzerland) was used as an outer phase lubricant during tableting.

Mercury (Sigma-Aldrich, Germany) was used for pressure intrusion porosimetry measurements as a non-wetting liquid.

*1.2 Methods*

*1.2.1 Roller compaction and milling*

Before roller compaction, each powder was sieved through a 1 000 $\mu$m sieve to destroy agglomerates. Excipients were blended with paracetamol to obtain relative paracetamol contents of 24.75% (w/w), 49.50% (w/w), 74.25% (w/w), and 99% (w/w). In addition, reference formulations without paracetamol were prepared. For comparative reasons, all the formulations contained 1wt.% of sodium stearyl fumarate as an inner lubricant. The powders were mixed for 10 min in a tumbling mixer (Turbula T2C, Willy A. Bachofen AG, Switzerland) at 32 rpm. Granulation was performed with a Fitzpatrick® Chilsonator IR220 roller compactor (The Fitzpatrick Company, USA). Pressure was set either to 5 bar or to 20 bar. The feed rate and the roll speed were adjusted to obtain a ribbon thickness of 0.6 mm. After roller compaction the ribbons were milled with a FitzMill® L1A (The Fitzpatrick Company, USA) at 500 rpm.

*1.2.2 Screening*

A screening was performed. For each formulation 10 g of the respective ribbon were put on a sieve with 500 $\mu$m mesh size. The sieve was shaken for 10 minutes with the help of a vibrating sieve tower (Vibro Retsch, Switzerland) at a shaking displacement of 1.5 mm. The remaining fraction (*RF*) was calculated with the help of the following equation:

$$RF \text{ wt.} - \% = 100 - \left( \frac{(m_{tot} - m_{res}) \times 100}{m_{tot}} \right)$$

where $m_{tot}$ is the total mass applied initially on the sieve (g) and $m_{res}$ is the residual amount on the sieve (g) after 10 minutes of shaking.

Formulations with a remaining fraction of more than 60% were considered as robust enough for the tableting process.

*1.2.3 Granule analysis*

To determine the fine fraction obtained with the roller compaction process, the product of the roller compaction process (ribbons including fines) was collected, weighed ($m_{tot}$), and sieved through a sieve with 180 μm mesh size. The sieving was carried out in that the corresponding product obtained from the roller compaction process was put on a sieve with 180 μm mesh size. The sieve was shaken for 10 minutes with the help of a vibrating sieve tower (Vibro Retsch, Switzerland) at a shaking displacement of 1.5 mm.

The particle size distribution analysis was performed with a vibrating sieve tower (Vibro Retsch, Switzerland). The sieving tower was shaken for 10 minutes at a shaking displacement of 1.5 mm. Steel wire screens (Vibro Retsch, 200 x 25 mm, Germany) were used with mesh sizes in the order of 180 μm, 250 μm, 355 μm, 500 μm, and 710 μm. The amount of powder remaining on each sieve was weighed. Granules with a size of less than 180 μm or more than 710 μm were excluded.

The fine fraction (*FF*) was calculated with the help of the following equation:

$$FF \text{ wt.-}\% = \frac{(m_{tot} - m_{res}) \times 100}{m_{tot}}$$

where $m_{tot}$ is the mass applied initially on the sieve (g) and $m_{res}$ is the residual amount on the sieve (g) after 10 minutes of shaking.

The granule fraction of the size of from 180 μm to 710 μm were combined and subjected to the following tests:

Flowability as well as bulk and tapped density were measured for selected granular fractions according to Ph Eur (European Pharmacopeia. 7. Aufl. Strasbourg (France): Council of Europe; 2011). For the flowability measurement a Mettler PM460 balance (Mettler Toledo, Switzerland) was used. Three different openings (5 mm, 7 mm, and 9 mm) were chosen.

Bulk and tapped density were measured with a STAV 2003 volumeter (J. Engelsmann, Germany).

The Hausner ratio (tapped density/bulk density) was calculated according to Ph Eur (European Pharmacopeia. 7. Aufl. Strasbourg (France): Council of Europe; 2011).

*1.2.4 Tablet preparation*

For the tablet preparation, all granules with a size between 180 μm and 710 μm were used. The granules were dried overnight (12-15 h) under nitrogen flow in a vacuum drying cabinet of type KVTS 11 (Salvis, Switzerland) before they were mixed in a tumbling mixer (Turbula T2C, Willy A. Bachofen AG, Switzerland) for 10 minutes at 32 rpm. The granules were compressed with a 10 mm round, flat tooling, using a Styl'One 105 ml tablet press (Medel'Pharm, France). The tablet press was instrumented with the Analis software version 2.01 (Medel'Pharm, France). During compaction, tablet volume was kept constant at the lowest compressive pressure due to highly variable porosities of the raw materials. The punch gap was adjusted for each formulation to obtain a tablet height of 6 mm at a compressive pressure of 6.37 MPa (0.50 kN). The punch gap was kept constant over the whole compressive pressure range for each formulation. The formulations were compacted in triplicate at 10 different compressive pressures, ranging from 6 MPa to 500 MPa. If lamination occurred below 500 MPa, further increase of the compressive pressure was halted. Punches and die were manually lubricated with magnesium stearate. Tablets were compressed at minimum possible dwell time by setting the rise and fall of the lower punch to 750 ms and the "maintain" phase to 0 ms.

Temperature and relative humidity during tablet preparation and analysis were between 22.3°C and 25.9°C, and 28.5% and 50.0%, respectively.

*1.2.5 Tablet analysis (weight, dimensions, height, crushing force, tensile strength, and friability)*

Tablet weight, dimensions, height, and crushing force were measured in triplicate directly after tablet compression. Weight was determined with a Delta Range AX204 balance (Mettler Toledo, Switzerland). Diameter and height were measured with a micrometer screw of type CD-15CPX (Mitutoyo, Japan). Crushing forces below 400 N were measured with a tablet hardness tester (8M, Dr. Schleuniger Pharmatron, Switzerland) at a speed of impact of 1 mm/s. Harder tablets (> 400 N) were tested with the Styl'One tablet press (Medel'Pharm, France) as described in Stirnimann T, et al., Compaction of functionalized calcium carbonate, a porous and crystalline microparticulate material with a lamellar surface. International Journal of Pharmaceutics. 15. May2014;466(1-2):266-75.

Tensile strength was calculated with the following equation:

$$\sigma_t = \frac{2F}{\pi dh}$$

where $\sigma_t$ is the radial tensile strength (MPa), F is the crushing force (N), $d$ is the tablet diameter (mm), and $h$ is the tablet thickness (mm).

Friability of tablets compressed at 100 MPa was measured with a drum (Erweka, Germany) according to Ph Eur (European Pharmacopeia. 7. Aufl. Strasbourg (France): Council of Europe; 2011).

*1.2.6 Heckel and modified Heckel equation*

With the help of the software OriginPro version 8.5 (OriginLab Corporation, USA), the Heckel expression (see also Heckel RW. Density-pressure relationships in powder compaction. Trans Metall Soc AIME. 1961;221:671-5 and Heckel RW. An analysis of powder compaction phenomena. Trans Metall Soc AIME. 1961;221:1001-8) was fitted according to the following equation:

$$\ln\left(\frac{1}{1-\rho}\right) = k\sigma + A$$

where p is the tablet density (g/cm$^3$), $k$ is the Heckel parameter (MPa$^{-1}$), $\sigma$ is the compressive pressure (MPa), and A is a constant. 4 data points between 50 MPa and 200 MPa were considered for the Heckel analysis.

The density (p) was calculated according to the following equation:

$$\rho = \frac{(m/\pi r^2 h)}{\rho_{\text{true}}}$$

where $m$ is the tablet mass (g), $r$ is the tablet radius (cm), h is the tablet height (cm), and $\rho_{\text{true}}$ is the true density of the skeletal forming substance within the porous tablet (g/cm$^3$).

Mean yield pressure ($\sigma_y$, MPa) was calculated with the following equation:

$$\sigma_y = \frac{1}{k}$$

where k is the Heckel parameter (MPa$^{-1}$).

The modified Heckel equation (see also M. Kuentz et al., Pressure susceptibility of polymer tablets as a critical property: A modified Heckel equation. Journal of Pharmaceutical Sciences. 1999;88(2):174-9) was fitted according to the following equation:

$$\sigma = \frac{1}{C}\left[\rho_{rc} - \rho - (1 - \rho_{rc})\ln\left(\frac{1-\rho}{1-\rho_{rc}}\right)\right]$$

where $\sigma$ is the compressive pressure (MPa), C is a constant (MPa$^{-1}$), $\rho_{rc}$ is the relative critical density, and p is the relative density of the tablet. For the modified Heckel analysis 6 data points between 5 MPa and 200 MPa were considered.

*1.2.7 Pore size distribution in tablets*

To measure the pore size distribution, tablets were prepared as described in section 1.2.4. The analysis was performed with an Auto Pore IV 9500 mercury porosimeter (Micromeritics, USA). Low-pressure and high-pressure mercury intrusion ranged from 3.59 kPa to 206.64 kPa and from 206.78 kPa to 206.78 MPa, respectively. Equilibration time was set to 10 s over the whole pressure range.

**Results and Discussion**

*2.1 Screening*

For tableting, the granules should preferably show certain rigidity for further processing. The fine fraction (< 500 µm) gives a hint on the rigidity of a ribbon. A screening with 18 formulations yielded 10 formulations of interest because they all showed a remaining fraction (> 500 µm) of more than 60% (w/w). This means that only 40% of the solid (in the form of ribbons) was collapsed into fines with a size of less than 500 µm after shaking for 10 minutes.

FCC with SSF showed a very high remaining fraction (approx. 90%) during roller compaction, independent of the applied roll pressure (the value for the remaining fraction for granules prepared at 20 bar is within the standard deviation of the value for 5 bar). This result showed that rigid ribbons made of FCC could be roller-compacted even at low pressures (5 bar). In general, higher remaining fractions were observed; thus, more rigid ribbons for the powder blends that were roller compacted at higher pressure (20 bar). This effect was more pronounced for mixtures containing the microcrystalline cellulose Avicel. Avicel with SSF admixtures processed at 5 bar roll pressure showed only 10% (w/w) remaining fraction, which is a sign of very fragile ribbon. It is thus assumed that, in contrast to FCC, the pressure of 5 bar was not enough for Avicel to form a sufficient number of interparticle bonds. At 20 bar the Avicel and SSF powder blend did not enter the compression region and resulted in the failure within the feeding zone of the roller compactor.

*2.2 Granule properties*

Table 2 shows the fine fraction during roller compaction for prepared formulations. It can be gathered that FCC mixtures showed a 50% reduction of the fine fraction after increasing compressive pressure during roller compaction from 5 bar to 20 bar. Comparable to the microcrystalline cellulose of the prior art (Avicel), higher fine fractions were observed for FCC by increasing the amount of paracetamol in the mixtures. However, the fine fraction at 74.25% drug load was much lower than observed for the prior art microcrystalline cellulose (Avicel).

By employing roller compaction of FCC it was possible to increase the flowability of FCC significantly. With respect to flowability, table 1 shows that at the same drug load and at the same compressive pressure during roller compaction, the FCC mixtures were always faster than the mixtures of the prior art (comprising Avicel).

Table 2 further shows that 5 bar compacted granules showed lower densities compared to granules compacted at 20 bar due to a looser packing derived from the lower applied pressure during roller compaction. In general, densities for FCC mixtures were always higher compared to Avicel mixtures.

The Hausner ratio lies between 1.04 and 1.11 for all the mixtures, meaning that the consolidation during tapping was minimal. It is noted that the Hausner ratio became smaller by increasing the amount of paracetamol. A possible reason is the structure and smooth surface texture of paracetamol, which does not allow rearrangement after the initial packing. Lamellae of FCC and the fibres of Avicel can interlock and prevent the particles from forming a dense packing. The higher the amount of paracetamol in the formulation, the smaller the interlocking effects of Avicel and FCC, hence the reduction of the Hausner ratio.

*2.3 Tablet properties*

Table 2 shows the resulting tablet weights for different formulations. Granules roller-compacted at 5 bar yielded lighter tablets than the granules at 20 bar due to less densification during roller compaction. Among paracetamol mixtures processed at 20 bar, the FCC-paracetamol mixtures produced heavier tablets compared to Avicel-paracetamol mixtures at the same drug-excipient ratio due to higher FCC bulk density (ca. 0.64 g/cm$^3$) compared to the microcrystalline cellulose Avicel of the prior art (ca. 0.57 g/cm$^3$).

Table 2 also shows that the tablet friability was considerably lower for FCC-paracetamol mixtures than for Avicel-paracetamol blends. Among the microcrystalline cellulose Avicel formulations, friability could only be measured for the tablets with 49.50 % drug load. It is assumed that the elastic properties of paracetamol could not be compensated by Avicel and, as a result, the tablets did not show enough physical strength to pass the friability test.

Friability values for FCC with admixtures of sodium stearyl fumarate (independent of pressure applied during roller compaction) and FCC in combination with a low drug load (roller compacted at 5 bar) were below 1 %. Furthermore, FCC granules obtained at 5 bar produced tablets with significantly lower friability than the ones compressed at 20 bar. This result is also explained in Figure 1.

Figure 1 shows the tablet tensile strengths of FCC-based tablet formulations as a function of compressive pressures. Up to 250 MPa pressure, the tensile strength increased almost linearly for all formulations at increasing compressive pressure. In particular, the friability is dependent on the tablet tensile strength, which is a function of surface available for contacts. The higher the pressure during roller compaction, the denser is the packing of the particles and the higher the loss of surface area.

At very high pressures (~ 400 MPa), tensile strength of tablets made of 5 bar granules and 20 bar granules were comparable. At such stresses, the granules (and also the particles within a granule) could break and, hence, provide additional surface area for bond-making contacts. For granules compressed at 20 bar, more compressive pressure was necessary to break the particles, in contrast to granules obtained at 5 bar. In contrast to tablets made of powder blends, the risk for tablet lamination was reduced at higher compressive pressures using FCC granules.

The positive effect of high specific surface on the stability of tablets is supported by tablet tensile strength results for FCC-paracetamol and Avicel-paracetamol mixtures. Figure 2 shows the tensile strength as a function of compressive pressure for paracetamol mixtures with FCC and Avicel. The mixtures with FCC reached much higher tensile strength values than the mixtures of Avicel and paracetamol, except the formulation with 24.75 % FCC. For the mixture with 74.25 % paracetamol, FCC reached the maximal tensile strength (0.16 MPa) already at 50 MPa. The Avicel formulation with the same drug load reached this value after 150 MPa compressive pressure was applied. The Avicel formulation with 74.25 % drug load reached a 3.4 times higher tensile strength than FCC in combination with paracetamol, but needed 10 times more pressure to reach this value. At higher drug load, FCC showed superior properties compared to Avicel, especially in the lower pressure range.

Table 3 shows the values for the Heckel and modified Heckel analysis. The higher the pressures during roller compaction, the lower the values for the Heckel parameter ($k$) for the tablets. A low value for "$k$" indicates higher pressure is needed to obtain a stable tablet at the same tensile strength. The values for relative critical density ($\rho_{rc}$), i.e. the tablet density at which the stable compacts can be first formed, supported the results from the Heckel analysis.

*2.4 Mercury porosimetry*

Figure 3 shows a mercury porosimetry plot of the pore size distributions of FCC tablets made with powder and granulates. The tablets were compressed at three different compressive pressures. By increasing the compressive pressure, the characteristic peaks were shifted to smaller pore diameters (Figure 3) as a result of FCC densification and collapsing of lamellae on the surface of particles. The plots of tablets compressed at the same compressive pressure were almost congruent, for all types of the primary material (powder or granules). The only noted difference is the higher pore volume of granules at larger pore diameters compared to powders. The intraparticular structure of FCC (pores with a diameter of less than 0.02 $\mu$m) was affected neither by the roller compaction process, nor by tableting.

Table 2: Granule and tablet properties. "x" is indicating that no tablet was obtained at 100 MPa (not stable enough / lamination). "Fail" means that the sample failed the test due to broken tablets.

| Mixtures | Fine fraction (%) | Flowability | (g/s) | | Bulk density (g/cm$^3$) | Tapped density (g/cm$^3$) | Hausner ratio | Tablet weight (mg) | Friability at 100 MPa (%) |
|---|---|---|---|---|---|---|---|---|---|
| | | 5 mm | 7 mm | 9 mm | | | | | |
| 99 % FCC + 1 % SSF_5 bar | 12 | 5.10 ± 0.05 | 2.93 ± 0.06 | 1.19 ± 0.01 | 0.53 | 0.56 | 1.06 | 350.1 ± 2.6 | 0.24 |
| 99 % FCC + 1 % SSF 20 bar | 6 | 8.51 ± 0.02 | 4.72 ± 0.01 | 1.91 ± 0.01 | 0.79 | 0.82 | 1.04 | 462.6 ± 4.2 | 0.73 |
| 74.25 % FCC + 24.75 % Paracetamol + 1 % SSF_5 bar | 21 | 5.52 ± 0.04 | 3.09 ± 0.00 | 1.27 ± 0.02 | 0.55 | 0.58 | 1.06 | 371.4 ± 2.9 | 0.56 |
| 74.25 % FCC + 24.75 % Paracetamol + 1 % SSF 20 bar | 10 | 7.54 ± 0.02 | 4.19 ± 0.01 | 1.68 ± 0.02 | 0.66 | 0.73 | 1.11 | 436.7 ± 3.7 | 1.13 |
| 49.50 % FCC + 49.50 % Paracetamol + 1 % SSF 20 bar | 30 | 7.02 ± 0.05 | 3.85 ± 0.02 | 1.56 ± 0.01 | 0.63 | 0.68 | 1.07 | 442.0 ± 4.3 | 1.76 |

(continued)

| Mixtures | Fine fraction (%) | Flowability | (g/s) | | Bulk density (g/cm³) | Tapped density (g/cm³) | Hausner ratio | Tablet weight (mg) | Friability at 100 MPa (%) |
|---|---|---|---|---|---|---|---|---|---|
| | | 5 mm | 7 mm | 9 mm | | | | | |
| 24.75 % FCC + 74.25 % Paracetamol + 1 % SSF 20 bar | 29 | 7.17 ± 0.02 | 3.96 ± 0.00 | 1.58 ± 0.02 | 0.64 | 0.68 | 1.05 | 435.2 ± 2.7 | x |
| 74.2 5 % Avicel + 24.75 % Paracetamol + 1 % SSF 20 bar | 8 | 5.98 ± 0.04 | 3.44 ± 0.02 | 1.41 ± 0.00 | 0.55 | 0.60 | 1.10 | 375.6 ± 3.4 | Fail |
| 49.50 % Avicel + 49.50 % Paracetamol + 1 % SSF 20 bar | 17 | 6.13 ± 0.02 | 3.41 ± 0.01 | 1.33 ± 0.01 | 0.56 | 0.61 | 1.09 | 383.5 ± 6.5 | 6.71 |
| 24.75 % Avicel + 74.25 % Paracetamol + 1 % SSF_20 bar | 45 | 6.19 ± 0.02 | 3.54 ± 0.03 | 1.39 ± 0.02 | 0.61 | 0.64 | 1.04 | 394.9 ± 4.2 | Fail |
| 99 % Paracetamol + 1 % SSF 20 bar | 55 | 6.39 ± 0.06 | 3.57 ± 0.02 | 1.49 ± 0.01 | 0.63 | 0.68 | 1.07 | 448.7 ± 2.1 | x |

Table 3: Heckel and modified Heckel parameters. The values for mixtures containing Avicel and paracetamol are not shown because some values were missing for the fitting in the chosen pressure range.

| Substances | Heckel equation | | | | Modified Heckel equation | | |
|---|---|---|---|---|---|---|---|
| | $k(10^{-3} MPa^{-1})$ ± SD | A ± SD | $\sigma_y$ (MPa) | $R^2$ | $C(10^{-3} MPa^{-1})$ ± SD | $\rho_{rc}$ ± SD | $R^2$ |
| 100 % FCC powder | 2.61 ± 0.15 | 0.44 ± 0.02 | 383 | 0.990 | 1.006 ± 0.064 | 0.160 ± 0.014 | 0.998004 |
| 99 % FCC + 1 %SSF_5 bar | 2.43 ± 0.15 | 0.43 ± 0.02 | 412 | 0.989 | 0.767 ± 0.031 | 0.193 ± 0.008 | 0.999036 |
| 99 % FCC + 1 %SSF_20 bar | 2.13 ± 0.08 | 0.55 ± 0.01 | 469 | 0.996 | 0.525 ± 0.029 | 0.303 ± 0.008 | 0.998171 |
| 75 % FCC + 25 % P powder | 2.97 ± 0.28 | 0.62 ± 0.04 | 336 | 0.974 | 1.051 ± 0.122 | 0.280 ± 0.024 | 0.994061 |
| 74.25 % FCC + 24.75 % P +1 % SSF_5 bar | 2.82 ± 0.13 | 0.58 ± 0.02 | 354 | 0.994 | 0.891 ± 0.021 | 0.285 ± 0.004 | 0.999704 |
| 74.25 % FCC + 24.75 % P +1 % SSF_20 bar | 2.29 ± 0.09 | 0.69 ± 0.01 | 437 | 0.995 | 0.549 ± 0.018 | 0.380 ± 0.004 | 0.999448 |
| 50 % FCC + 50% P powder | 3.23 ± 0.32 | 0.83 ± 0.04 | 309 | 0.972 | 0.985 ± 0.127 | 0.406 ± 0.023 | 0.993015 |
| 49.5 % FCC + 49.5 % P +1 % SSF_20 bar | 2.46 ± 0.21 | 0.95 ± 0.03 | 406 | 0.979 | 0.507 ± 0.043 | 0.509 ± 0.010 | 0.996791 |
| 25 % FCC + 25 % P powder | 3.19 ± 0.42 | 1.13 ± 0.05 | 313 | 0.951 | 0.708 ± 0.137 | 0.562 ± 0.025 | 0.98597 |

(continued)

| Substances | Heckel equation | | | | Modified Heckel equation | | |
|---|---|---|---|---|---|---|---|
| | $k(10^{-3}MPa^{-1}) \pm SD$ | $A \pm SD$ | $\sigma_y$ (MPa) | $R^2$ | $C(10^{-3} MPa^{-1}) \pm SD$ | $\rho_{rc} \pm SD$ | $R^2$ |
| 24.75 % FCC + 74.25 % P +1 % SSF 20 bar | nd | nd | nd | nd | nd | nd | nd |

**Conclusion**

Roller compaction of FCC revealed rigid ribbons at a low fine fraction, even at low pressures during roller compaction. The resulting granules made of FCC and a pharmaceutically active agent showed better flowabilities than corresponding microcrystalline cellulose (Avicel) based formulations. In addition, tablets composed of granulated FCC and a pharmaceutically active agent showed significantly higher tensile strengths over the whole pressure range than microcrystalline cellulose (Avicel) based formulations. Thus, granulated formulations of FCC have superior properties with respect to flowability and compactability as compared to microcrystalline cellulose (Avicel) based formulations or non-granulated FCC formulations.

**Claims**

1. A method for producing a pharmaceutical delivery system, comprising the steps of:

   a) providing surface-reacted calcium carbonate, which is a reaction product of natural ground or precipitated calcium carbonate with carbon dioxide and one or more acids in an aqueous medium, wherein the carbon dioxide is formed in situ by the acid treatment and/or is supplied from an external source;
   b) providing at least one pharmaceutically active agent or pharmaceutically inactive precursor thereof;
   c) providing at least one formulating aid;
   d) mixing the surface-reacted calcium carbonate of step a), the at least one pharmaceutically active agent or pharmaceutically inactive precursor thereof of step b) and the at least one formulating aid of step c); and
   e) compacting the mixture obtained in step d) by means of a roller compacter at a compaction pressure in the range from 4 to 20 bar; and
   f) compacting the roller compacted mixture obtained in step e) for obtaining the pharmaceutical delivery system.

2. The method of claim 1, wherein the natural ground calcium carbonate is selected from calcium carbonate containing minerals selected from the group comprising marble, chalk, dolomite, limestone and mixtures thereof; or the precipitated calcium carbonate is selected from the group comprising precipitated calcium carbonates having aragonitic, vateritic or calcitic mineralogical crystal forms and mixtures thereof.

3. The method according to any one of claims 1 or 2, wherein the surface-reacted calcium carbonate

   a) has a BET specific surface area of from 20.0 $m^2$/g to 200.0 $m^2$/g, preferably from 20.0 $m^2$/g to 180.0 $m^2$/g, more preferably from 30.0 $m^2$/g to 160.0 $m^2$/g, even more preferably from 40.0 $m^2$/g to 150.0 $m^2$/g, and most preferably from 50.0 $m^2$/g to 140.0 $m^2$/g, measured using nitrogen and the BET method according to ISO 9277; and/or
   b) comprises particles having a volume median grain diameter $d_{50}$ of from 2.0 to 50.0 $\mu$m, preferably from 2.5 to 25.0 $\mu$m, more preferably from 2.8 to 20.0 $\mu$m, even more preferably from 3.0 to 10.0 $\mu$m, and most preferably from 4.0 to 8.0 $\mu$m; and/or
   c) has an intra-particle intruded specific pore volume within the range of 0.15 to 1.35 $cm^3$/g, preferably of 0.30 to 1.30 $cm^3$/g, and most preferably of 0.40 to 1.25 $cm^3$/g, calculated from a mercury intrusion porosimetry measurement.

4. The method according to any one of claims 1 to 3, wherein the at least one pharmaceutically active agent or pharmaceutically inactive precursor thereof is selected from the group comprising pharmaceutically active agent or pharmaceutically inactive precursor of synthetic origin, semi-synthetic origin, natural origin and combinations thereof.

**5.** The method according to any one of claims 1 to 4, wherein the at least one formulating aid

a) is at least one inner-phase lubricant and/or outer-phase lubricant; and/or
b) is provided in a total amount from about 0.1 wt.-% to about 10.0 wt.-%, preferably from about 0.3 wt.-% to about 5.0 wt.-%, more preferably from about 0.5 wt.-% to about 2.5 wt.-% based on the total weight of the pharmaceutical delivery system.

**6.** The method according to any one of claims 1 to 5, wherein roller compacting step e) is carried out at a roller compaction pressure in the range from 4 to 15 bar, more preferably in the range from 4 to 10 bar and most preferably in the range from 4 to 7 bar.

**7.** The method according to any one of claims 1 to 6, wherein compacting step f) is a pelletizing or tableting step.

**8.** The method according to any one of claims 1 to 7, wherein the roller compacted mixture obtained in roller compacting step e) is subjected to a milling step before compacting step f) is carried out.

**9.** The method according to any one of claims 1 to 8, wherein the roller compacted mixture has a grain size of from 180 to 710 $\mu$m obtained by sieving on different mesh sizes, preferably with mesh sizes of 180 $\mu$m, 250 $\mu$m, 355 $\mu$m, 500 $\mu$m and 710 $\mu$m.

**10.** The method according to any one of claims 1 to 9, wherein the pharmaceutical delivery system

a) has a friability of $\leq 1.10$ %, measured according to Ph.Eur.4 on tablets compressed at 100 MPa; and/or
b) fulfils Formula (I)

$$(FR_{RCPh}) / (FR_{RCPl}) \geq 1.2 \qquad (I)$$

wherein

($FR_{RCPh}$) is the friability (in %), measured according to Ph.Eur.4, of a pharmaceutical delivery system RCPh, wherein the compaction pressure in step e) is at least 15 bar;
($FR_{RCPl}$) is the friability (in %), measured according to Ph.Eur.4, of a pharmaceutical delivery system RCPl, wherein the compaction pressure in step e) is less than the compaction pressure used for obtaining the pharmaceutical delivery system RCPh.

**11.** The method according to any one of claims 1 to 10, wherein the pharmaceutical delivery system fulfils Formula (II)

$$(FL_{RCP}) / (FL_{reference}) \geq 1.1 \qquad (II)$$

wherein

($FL_{RCP}$) is the flowability (in g/s), measured according to Ph.Eur.4 at an opening diameter of 7 mm, of the pharmaceutical delivery system;
($FL_{reference}$) is the flowability (in g/s), measured according to Ph.Eur.4 at an opening diameter of 7 mm, of the same pharmaceutical delivery system, in which the surface-reacted calcium carbonate is replaced by microcrystalline cellulose.

**12.** The method according to any one of claims 1 to 11, wherein the pharmaceutical delivery system is a tablet, mini-tablet, capsule or pellet.

**13.** Use of a surface-reacted calcium carbonate for improving the friability of a pharmaceutical delivery system, wherein the surface-reacted calcium carbonate is a reaction product of natural ground or precipitated calcium carbonate with carbon dioxide and one or more acids in an aqueous medium, wherein the carbon dioxide is formed in situ by the acid treatment and/or is supplied from an external source, wherein the pharmaceutical delivery system

a) has a friability of ≤ 1.10 %, measured according to Ph.Eur.4 on tablets compressed at 100 MPa; and/or
b) fulfils Formula (II)

$$(FR_{RCPh}) / (FR_{RCPl}) \geq 1.2 \qquad (I)$$

wherein

$(FR_{RCPh})$ is the friability (in %), measured according to Ph.Eur.4, of a pharmaceutical delivery system RCPh, wherein the compaction pressure in step e) is at least 15 bar;
$(FR_{RCPl})$ is the friability (in %), measured according to Ph.Eur.4, of a pharmaceutical delivery system RCPl, wherein the compaction pressure in step e) is less than the compaction pressure used for obtaining the pharmaceutical delivery system RCPh.

14. Use of a surface-reacted calcium carbonate for improving the flowability of a pharmaceutical delivery system, wherein the surface-reacted calcium carbonate is a reaction product of natural ground or precipitated calcium carbonate with carbon dioxide and one or more acids in an aqueous medium, wherein the carbon dioxide is formed in situ by the acid treatment and/or is supplied from an external source, wherein the pharmaceutical delivery system fulfils Formula (II)

$$(FL_{RCP}) / (FL_{reference}) \geq 1.1 \qquad (II)$$

wherein

$(FL_{RCP})$ is the flowability (in g/s), measured according to Ph.Eur.4 at an opening diameter of 7 mm, of the pharmaceutical delivery system;
$(FL_{reference})$ is the flowability (in g/s), measured according to Ph.Eur.4 at an opening diameter of 7 mm, of the same pharmaceutical delivery system, in which the surface-reacted calcium carbonate is replaced by microcrystalline cellulose.

15. A pharmaceutical delivery system, preferably a tablet, mini-tablet, capsule or pellet, obtained by the method according to any one of claims 1 to 12.

Fig. 1

Fig. 2

Fig. 3

Europäisches
Patentamt

European
Patent Office

Office européen
des brevets

# EUROPEAN SEARCH REPORT

**Application Number**

EP 14 19 9037

## DOCUMENTS CONSIDERED TO BE RELEVANT

| Category | Citation of document with indication, where appropriate, of relevant passages | Relevant to claim | CLASSIFICATION OF THE APPLICATION  (IPC) |
|---|---|---|---|
| X | TANJA STIRNIMANN ET AL:  "Compaction of functionalized calcium carbonate, a porous and crystalline microparticulate material with a lamellar surface", INTERNATIONAL JOURNAL OF PHARMACEUTICS, vol. 466, no. 1-2, 1 May 2014 (2014-05-01) , pages 266-275, XP55179910, ISSN: 0378-5173, DOI: 10.1016/j.ijpharm.2014.03.027 | 1,3-5,7, 9-15 | INV. A61K9/20 A61K31/167 |
| Y | * the whole document * | 2,6,8 | |
| Y,D | EP 2 719 373 A1 (OMYA INT AG [CH]) 16 April 2014 (2014-04-16) * page 4, paragraph 10-11 * * page 5, paragraph 31-32 * * page 6, paragraph 41-44 * * page 7, paragraph 56-58 * * page 8, paragraph 59-75 * | 2,6,8 | |
| Y,D | EP 2 719 376 A1 (OMYA INT AG [CH]) 16 April 2014 (2014-04-16) * page 2, paragraph 1 * * page 3, paragraph 25 * * page 4, paragraph 28-40 * * page 5, paragraph 43-54 * * page 6, paragraph 63 * * pages 7-8, paragraph 79 * * page 8, paragraphs 80-86, 95 * | 2,6,8 | TECHNICAL FIELDS SEARCHED     (IPC) A61K |
| Y,D | EP 2 264 108 A1 (OMYA DEVELOPMENT AG [CH]) 22 December 2010 (2010-12-22) * the whole document * | 2 | |

The present search report has been drawn up for all claims

| Place of search | Date of completion of the search | Examiner |
|---|---|---|
| Munich | 14 April 2015 | Raposo, Antonio |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 14 19 9037

This annex lists the patent family members relating to the patent documents cited in the above-mentioned European search report.
The members are as contained in the European Patent Office EDP file on
The European Patent Office is in no way liable for these particulars which are merely given for the purpose of information.

14-04-2015

| Patent document cited in search report | | Publication date | Patent family member(s) | | Publication date |
|---|---|---|---|---|---|
| EP 2719373 | A1 | 16-04-2014 | AU | 2013328639 A1 | 02-04-2015 |
| | | | CA | 2884912 A1 | 17-04-2014 |
| | | | EP | 2719373 A1 | 16-04-2014 |
| | | | TW | 201438722 A | 16-10-2014 |
| | | | UY | 35071 A | 30-05-2014 |
| | | | WO | 2014057038 A1 | 17-04-2014 |
| EP 2719376 | A1 | 16-04-2014 | CA | 2885971 A1 | 17-04-2014 |
| | | | EP | 2719376 A1 | 16-04-2014 |
| | | | TW | 201427689 A | 16-07-2014 |
| | | | UY | 35070 A | 30-05-2014 |
| | | | WO | 2014057026 A1 | 17-04-2014 |
| EP 2264108 | A1 | 22-12-2010 | AT | 545682 T | 15-03-2012 |
| | | | AU | 2010261441 A1 | 02-02-2012 |
| | | | CA | 2765343 A1 | 23-12-2010 |
| | | | CN | 102459470 A | 16-05-2012 |
| | | | CO | 6440525 A2 | 15-05-2012 |
| | | | DK | 2264108 T3 | 29-05-2012 |
| | | | EP | 2264108 A1 | 22-12-2010 |
| | | | EP | 2443202 A1 | 25-04-2012 |
| | | | ES | 2382628 T3 | 12-06-2012 |
| | | | HR | P20120396 T1 | 30-06-2012 |
| | | | JP | 5584290 B2 | 03-09-2014 |
| | | | JP | 2012530042 A | 29-11-2012 |
| | | | KR | 20120037468 A | 19-04-2012 |
| | | | NZ | 597478 A | 21-12-2012 |
| | | | PL | 2264108 T3 | 31-07-2012 |
| | | | PT | 2264108 E | 23-05-2012 |
| | | | RS | 52297 B | 31-12-2012 |
| | | | RU | 2012101307 A | 27-07-2013 |
| | | | SI | 2264108 T1 | 29-06-2012 |
| | | | TW | 201105583 A | 16-02-2011 |
| | | | US | 2012186492 A1 | 26-07-2012 |
| | | | UY | 32708 A | 31-01-2011 |
| | | | WO | 2010146530 A1 | 23-12-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3976764 A
- DE 3527852 A1
- EP 0338861 A2
- EP 0717988 A1
- US 4451260 A
- US 4814179 A
- EP 2719376 A1
- EP 2719373 A1
- FR 2787802 B1
- WO 0039222 A1
- US 20040020410 A1
- WO 2004083316 A1
- WO 2005121257 A2
- WO 2009074492 A1
- EP 2264108 A1
- WO 2010146530 A1
- EP 2264109 A1
- WO 2010146531 A1
- WO 2014001063 A
- WO 0039222 A
- WO 2004083316 A
- WO 2005121257 A
- WO 2009074492 A
- EP 2264108 A
- EP 2264109 A
- US 20040020410 A
- EP 2070991 A
- US 4627977 A, Gaffar
- US 4985459 A, Sunshine
- US 20030157213 A1
- US 20030206993 A1
- US 20030099741 A1

**Non-patent literature cited in the description**

- **PAWAR et al.** Gastroretentive dosage forms: A review with special emphasis on floating drug delivery systems. *Drug Delivery.,* February 2011, vol. 18 (2), 97-110
- **HARRIS, D. C.** Quantitative Chemical Analysis. W.H. Freeman & Co, 1991
- *European Pharmacopeia. 7. Aufl. Strasbourg (France): Council of Europe,* 2011
- European Pharmacopeia. Ph Eur. 2011
- **STIRNIMANN T et al.** Compaction of functionalized calcium carbonate, a porous and crystalline microparticulate material with a lamellar surface. *International Journal of Pharmaceutics,* 15 May 2014, vol. 466 (1-2), 266-75
- **HECKEL RW.** Density-pressure relationships in powder compaction. *Trans Metall Soc AIME,* 1961, vol. 221, 671-5
- **HECKEL RW.** An analysis of powder compaction phenomena. *Trans Metall Soc AIME,* 1961, vol. 221, 1001-8
- **M. KUENTZ et al.** Pressure susceptibility of polymer tablets as a critical property: A modified Heckel equation. *Journal of Pharmaceutical Sciences,* 1999, vol. 88 (2), 174-9